# EUROPÄISCHE PATENTSCHRIFT

(11) **EP 2 931 233 B1**
(45) Veröffentlichungstag und Bekanntmachung des Hinweises auf die Patenterteilung: **28.03.2018**
(21) Anmeldenummer: 13815413.3
(22) Anmeldetag: 10.12.2013
(51) Int. Cl.: A61K 8/41, A61K 8/60, A61Q 5/10

(54) **REDUZIERUNG DER AUTOXIDATION VON MITTELN ZUM OXIDATIVEN FÄRBEN UND/ODER AUFHELLEN VON KERATINFASERN**
REDUCTION OF THE AUTOXIDATION OF AGENTS FOR THE OXIDATIVE DYEING AND/OR BLEACHING OF KERATIN FIBERS
RÉDUCTION DE L'AUTOXYDATION D'AGENTS DESTINÉS À LA COLORATION OXYDATIVE ET/OU À L'ÉCLAIRCISSEMENT OXYDATIF DE FIBRES KÉRATINIQUES

(30) Priorität: 14.12.2012 DE 102012223207
(43) Veröffentlichungstag der Anmeldung: 21.10.2015
(73) Patentinhaber: Henkel AG & Co. KGaA, 40589 Düsseldorf (DE)
(72) Erfinder: NEUBA, Constanze, 41516 Grevenbroich (DE); JANSSEN, Frank, 51103 Köln (DE)
(86) Internationale Anmeldenummer: PCT/EP2013/076050
(87) Internationale Veröffentlichungsnummer: WO 2014/090784

(56) Entgegenhaltungen:
- WO-A2-2004/000248
- WO-A2-2012/095394
- DE-A1-102005 005 199
- JP-A- 2005 179 191
- JP-A- 2011 037 750
- KR-A- 20090 027 985
- US-A- 3 488 138
- US-A- 4 875 902
- US-A- 5 716 418
- US-A1- 2009 246 160
- DATABASE GNPD [Online] MINTEL; 1. September 2010 (2010-09-01), "Hair Sunscreen Colour Protect Cream", XP002722453, Database accession no. 1398195

## Beschreibung

Gegenstand der vorliegenden Erfindung sind Mittel zum Färben und/oder Aufhellen von keratinischen Fasern, insbesondere menschlichen Haaren, enthaltend in einem kosmetischen Träger eine spezielle Kombination aus zwei verschiedenen Alkylglucosiden mit unterschiedlicher Alkylkettenlänge, einem Oxidationsfarbstoffvorprodukt vom Entwicklertyp und einem Oxidationsfarbstoffvorprodukt vom Kupplertyp. Die Verwendung dieser speziellen Kombination zur Verhinderung der durch atmosphärischen Sauerstoff hervorgerufenenen Verfärbung in oxidativen Mitteln zum Färben und/oder Aufhellen der Keratinfasern ist ebenfalls Gegenstand der Erfindung.

Zur Bereitstellung farbverändernder kosmetischer Mittel, insbesondere für keratinische Fasern, wie beispielsweise Haare, kennt der Fachmann je nach Anforderung an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Die Oxidationsfärbemittel zeichnen sich durch hervorragende, lang anhaltende Färbeergebnisse aus. Neben der Färbung ist auch das Aufhellen bzw. Blondieren der eigenen Haarfarbe der ganz spezielle Wunsch vieler Verbraucher. Dazu werden die die Faser färbenden natürlichen oder künstlichen Farbstoffe meist oxidativ unter Einsatz von entsprechenden Oxidationsmitteln, wie beispielsweise Wasserstoffperoxid, entfärbt.

Üblicherweise werden oxidative Färbemittel in Form eines aus zwei Komponenten bestehenden "Kits" (Mehrkomponenten-Verpackungseinheit) angeboten, wobei die erste Komponente die Oxidationsfarbstoffvorprodukte und ein Alkalisierungsmittel (beispielsweise Ammoniak) und die zweite Komponenten das Oxidationsmittel enthält. Als Oxidationsmittel werden in der Regel Peroxide wie beispielsweise Wasserstoffperoxid eingesetzt. Oft ist zusätzlich noch ein Conditioner als dritte Komponente im Kit enthalten, der nach dem eigentlichen Färbevorgang zur Verbesserung des Pflegezustandes der Haare angewendet wird

Die Oxidationsfarbstoffvorprodukte (Entwickler und Kuppler) selbst sind nicht gefärbt, sondern die Bildung der eigentlichen Farbstoffe erfolgt erst im Verlauf der Anwendung durch den Kontakt der Oxidationsfarbstoffvorprodukte mit dem Oxidationsmittel (Wasserstoffperoxid). In chemischer Reaktion werden die als Oxidationsfarbstoffvorprodukte eingesetzten Entwickler (wie beispielsweise p-Phenylendiamin-Derviate oder p-Aminophenolderivate) durch Wasserstoffperoxid zunächst oxidativ in eine reaktive Zwischenstufe, auch Chinonimin oder Chinondiimin genannt, überführt, welche dann in einer oxidativen Kupplungsreaktion mit den Kupplern zum jeweiligen Farbstoff reagiert.

Da die Entwickler und Kuppler oxidativ zunächst in reaktive Zwischenstufen und im Anschluss daran in Farbstoffe überführt werden, sind sie naturgemäß gegenüber Oxidationsmitteln hochempfindlich. Von zentralem Erfolg für die oxidative Keratinfärbung ist es, dass die Bildung der Farbstoffe gezielt durch Wasserstoffperoxid und erst während des Färbevorgangs innerhalb der keratinischen Faser erfolgt. Eine frühzeitige Reaktion von Entwicklern mit Kupplern führt zu einer vorzeitigen Farbstoffbildung, so dass die Farbstoffvorprodukte für die eigentliche Färbung innerhalb der Keratinfasern nicht mehr - oder nicht mehr in ausreichender Menge - zur Verfügung stehen. Zur Vermeidung dieser vorzeitigen Reaktion von Entwicklern und Kupplern werden die Oxidationsfarbstoffvorprodukte daher üblicherweise vom Oxidationsmittel getrennt in verschiedenen kosmetischen Trägern konfektioniert.

Es ist ein seit langem bekanntes Problem in der Haarfärbepraxis, dass die Ausbildung der Farbstoffe nicht nur durch das gezielt zugesetzte Oxidationsmittel initiiert wird, sondern dass auch der in der Atmosphäre enthaltene Luftsauerstoff gegenüber Entwicklern und Kupplern als Oxidationsmittel wirkt.

Tritt eine Farbcreme, welche Entwickler, Kuppler und aller Regel nach zusätzlich Alkalisierungsmittel enthält, mit atmosphärischem Sauerstoff in Kontakt, so wird die Farbstoffbildung durch den Luftsauerstoff bereits vor Beginn des eigentlichen Färbevorgangs initiiert. Dieser unerwünschte Vorgang wird auch als Autoxidation der Farbstoffvorprodukte bezeichnet. Aufgrund unterschiedlicher Reaktionskinetiken führt die durch den Luftsauerstoff hervorgerufene Autoxidation der Oxidationsfarbstoffprodukte in vielen Fällen auch nicht zu den gewünschten Farbstoffen, sondern es werden andere unspezifische, wenig brillant gefärbte und deshalb unerwünschte Reaktionsprodukte gebildet.

Eine Folge der Autoxidation ist eine unkosmetisch anmutende, verfrühte Verfärbung der Farbcreme, die vom Anwender als höchst unattraktiv wahrgenommen wird. Eine weitere Konsequenz der zuvor beschriebenen Autoxidation ist eine Minderung der Färbeleistung des entsprechenden Färbemittels, da ein nicht unwesentlicher Anteil der Farbstoffvorprodukte durch den Luftsauerstoff vorzeitig abreagiert und auf diese Weise für den eigentlichen Färbevorgang nicht mehr zur Verfügung steht. Eine in der Literatur wohlbekannte Methode zur Minimierung der Autoxidation von Oxidationsfarbstoffvorprodukten ist der Einsatz von Reduktionsmitteln in den Farbcremes. Gängige geeignete Reduktionsmittel sind beispielsweise Natriumsulfit oder andere Alkali- oder Erdalkalisulfite, Vitamin C (Ascorbinsäure) und Dehydroascorbinsäure. Auch Thiolverbindungen wie Cystein, Thioglycolsäure oder Thiomilchsäure können zu Minimierung der Autoxidation in Farbcremes Anwendung finden, sind jedoch aufgrund der mit dieser Substanzklasse verbundenen geruchlichen Belastungen weniger erwünscht.

So wird beispielsweise in DE 10 2007 027862 A1 auf bereits abgefüllte Haarfärbemittel eine Lösung enthaltend ein Reduktionsmittel aufgebracht.

Der Einsatz von Reduktionsmitteln in Farbcremes kann jedoch auch mit Nachteilen behaftet sein: Genau wie die Oxidationsfarbstoffvorprodukte reagieren auch die Reduktionsmittel während der Anwendung mit dem Oxidationsmittel (Wasserstoffperoxid). Entsprechend kann Oxidationsmittel, welches mit den Reduktionsmitteln abreagiert, nicht mehr wie eigentlich gewünscht für den Haarfärbeprozess zur Verfügung stehen. Auf diese Weise kann auch der Einsatz großer Reduktionsmittelmengen zu einer Abschwächung der Farbleistung des Färbemittels führen.

WO 2012/095394 A2 offenbart ein Verfahren zum oxidativen Färben von Haaren, bei dem eine aus zwei Mitteln (a) und (b) hergestellt Anwendungsmischung auf die Haare appliziert wird. Hierbei kann das Mittel (A) C₈-C₃₀-Alkylglucoside enthalten.

US 5 716 418 A adressiert Färbe- und Aufhellmittel enthaltend Oxidationsfarbstoffvorprodukte und C₁₂-C₁₆-Alkylglycoside.

WO 2004/000248 A2 beschreibt kosmetische Zusammensetzungen, enthaltend ein spezielles Copolymer und nichtionischer Emulgatoren. Als nichtionische Emulgatoren können unter anderem C₈-C₂₂-Alkylmono- und oligoglycoside eingesetzt werden.

Eine optimale Methode zur möglichst vollständigen Verhinderung der Autoxidation von Oxidationsfarbstoffvorprodukten, ohne hierbei auf den Einsatz großer Mengen Reduktionsmittel zurückgreifen zu müssen, ist aus dem Stand der Technik bislang nicht bekannt.

Aufgabe der vorliegenden Erfindung war daher die Bereitstellung von Mitteln zum oxidativen Färben und/oder Aufhellen von Haaren, welche sich durch den Kontakt mit atmosphärischem Sauerstoff nicht bzw. möglichst wenig verfärben. Auch die nach Vermischen mit dem Oxidationsmittel resultierenden anwendungsbereiten Färbemittel sollten sich durch den Kontakt mit atmosphärischem Sauerstoff nicht bzw. möglichst wenig verfärben und ein kosmetisch und ästhetisch ansprechendes Erscheinungsbild behalten, ohne hierbei jedoch im Hinblick auf ihre Färbeleistung Einbußen zu erleiden.

Überraschenderweise hat sich im Zuge der zu dieser Erfindung führenden Arbeiten herausgestellt, dass es möglich ist, die Autoxidation von Oxidationsfarbstoffvorprodukten effektiv zu minimieren, wenn die Mittel neben den Oxidationsfarbstoffvorprodukten vom Entwicklertyp und vom Kupplertyp spezielle Kombinationen aus zwei verschiedenen Alkylglucosiden mit unterschiedlichen Alkylkettenlängen enthalten.

Ein erster Gegenstand der vorliegenden Erfindung ist ein anwendungsbereites Mittel zum Färben und/oder Aufhellen von keratinischen Fasern, dadurch gekennzeichnet, dass es unmittelbar vor der Anwendung durch Vermischen der Zubereitungen (A) und (B) hergestellt wird, wobei
- es sich bei der Zubereitung (A) um ein Mittel handelt, enthaltend in einem kosmetischen Träger
   (a) ein oder mehrere Alkylglucoside der Formel (I) in einer Gesamtmenge von 0,3 bis 4,5 Gew.-% - bezogen auf das Gesamtgewicht des anwendungsbereiten Mittels - 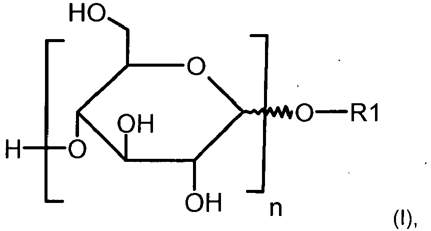
      worin R1 für eine unverzweigte oder verzweigte, gesättigte oder ungesättigte C₂₀-C₂₈-Alkylgruppe steht und
      n für eine ganze Zahl von 1 bis 10 steht,
   (b) mindestens ein zweites Alkylglucosid der Formel (II) 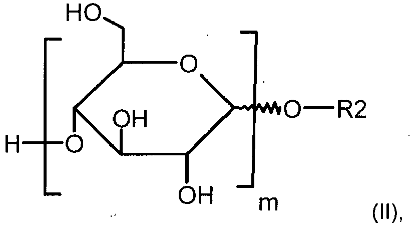
      worin R2 für eine unverzweigte oder verzweigte, gesättigte oder ungesättigte C₈-C₁₈-Alkylgruppe steht und
      m für eine ganze Zahl von 1 bis 10 steht,
   (c) mindestens ein Oxidationsfarbstoffvorprodukt vom Entwicklertyp und (d) mindestens ein Oxidationsfarbstoffvorprodukt vom Kupplertyp und es sich bei der Zubereitung (B) um ein Mittel handelt, das in einem kosmetischen Träger Wasserstoffperoxid enthält, und das durch Vermischen der Zubereitungen (A) und (B) hergestellte anwendungsbereite Mittel Wasserstoffperoxid in einer Menge von 0,5 bis 8,0 Gew.-% (berechnet als 100 %-iges Wasserstoffperoxid) - bezogen auf das Gesamtgewicht des anwendungsbereiten Mittels - enthält.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare. Unter dem erfindungsgemäß verwendeten Begriff "Mittel zum Färben und/oder Aufhellen" von Keratinfasern werden oxidative Färbemittel verstanden. Oxidative Färbemittel enthalten Oxidationsfarbstoffvorprodukte, sogenannte Entwickler und Kupplerkomponenten. Entwickler und Kuppler diffundieren getrennt in die Keratinfaser hinein und bilden unter dem Einfluss von einem Alkalisierungsmittel (z.B. Ammoniak) und einem Oxidationsmittel (meist Wasserstoffperoxid) in chemischer Reaktion miteinander die eigentlichen Farbstoffe aus. Abhängig von der Menge des eingesetzten Oxidationsmittels wird die Keratinfaser während der Färbung gleichzeitig mehr oder weniger stark aufgehellt, da das Oxidationsmittel nicht nur den Farbstoffbildungsprozess von Entwicklern und Kupplern initiiert, sondern auch die haareigenen Pigmente (Melanine) oxidativ zerstört. Je nach Einsatzmengen der Oxidationsfarbstoffvorprodukte und des Oxidationsmittels kann es sich bei der oxidativen Färbung daher vornehmlich um eine Färbung (mit hohem Farbstoffanteil) oder vornehmlich um eine Aufhellung (mit hohem Anteil an Oxidationsmittel) handeln. In letzterem Fall werden die Oxidationsfarbstoffvorprodukte hauptsächlich zur Nuancierung des Aufhellergebnisses eingesetzt. Die erfindungsgemäßen Mittel enthalten die erfindungswesentlichen Bestandteile in einem kosmetischen Träger, bevorzugt in einem geeigneten wässrigen, alkoholischen oder wässrigalkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Als ersten wesentlichen Formulierungsbestandteil enthalten die erfindungsgemäßen Zubereitungen (A) mindestens ein Alkylglucosid (a) der Formel (I) 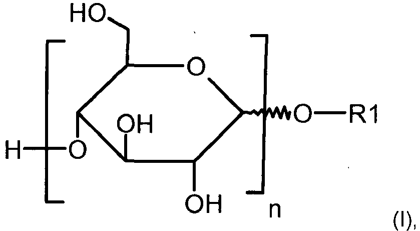 worin R1 für eine unverzweigte oder verzweigte, gesättigte oder ungesättigte C₂₀-C₂₈-Alkylgruppe steht und n für eine ganze Zahl von 1 bis 10 steht.

Unter einer unverzweigten Alkylgruppe wird eine lineare Alkylgruppe verstanden. Unter Alkylglucosiden werden im Sinne der vorliegenden Erfindung die Glycoside von Glucose verstanden, wobei die Ausbildung der glycosidischen Bindung in Form einer Kondensationsreaktion ausgehend von der anomeren Hydroxygruppe des Glucoserestes (vorliegend in seiner Pyranoseform) mit der alkoholischen OH-Gruppe eines C₂₀-C₂₈-Alkanols erfolgt. Die Ausbildung der glycosidischen Funktionalität erfolgt ausgehend von Glucose in ihrer Pyranoseform, wobei sowohl die Glucoside ausgehend von α-Glucose (Formel Ia) als auch von der β-Glucose (Formel Ib) von der Erfindung mit umfasst sind.

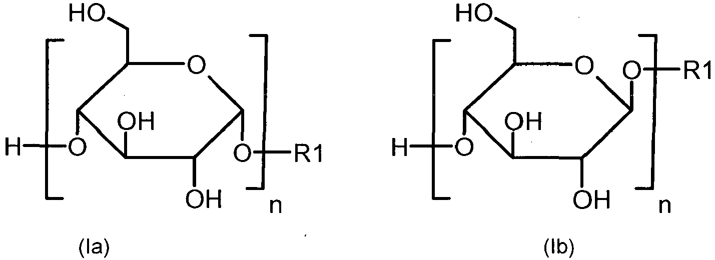

Der Rest R1 steht für eine unverzweigte oder verzweigte, gesättigte oder ungesättigte C₂₀-C₂₈-Alkylgruppe, und n steht für eine ganze Zahl von 1 bis 10.Es hat sich herausgestellt, dass die durch Luftsauerstoff hervorgerufene Autoxidation insbesondere dann wirkungsvoll minimiert werden kann, wenn der Rest R1 für eine unverzweigte, gesättigte C₂₀-C₂₄-Alkylgruppe, bevorzugt für eine unverzweigte, gesättigte C₂₀-C₂₂-Alkylgruppe und ganz besonders bevorzugt für eine unverzweigte, gesättigte C₂₀-Alkylgruppe steht.Weiterhin wird die erfindungsgemäße Aufgabenstellung insbesondere dann optimal gelöst, wenn n für eine Zahl von 1 bis 4, bevorzugt für die Zahlen 1 oder 2, und ganz besonders bevorzugt für die Zahl 1 steht.Ganz besonders bevorzugt wird als Alkylglucosid der Formel (I) die Verbindung (Ic) eingesetzt.

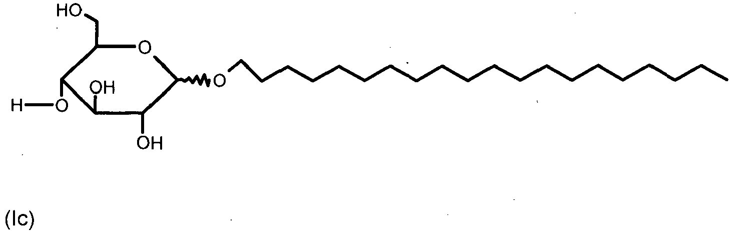

In einer besonders bevorzugten Ausführungsform ist ein anwendungsbereites Mittel zum Färben und/oder Aufhellen von keratinischen Fasern deshalb dadurch gekennzeichnet, dass die Zubereitung (A) als erstes Alkylglucosid (a) mindestens eine Verbindung der Formel (I) enthält, bei welcher
R1 für eine unverzweigte, gesättigte C₂₀-Alkylgruppe steht und n für die Zahl 1 steht.

Ein entsprechendes besonders bevorzugtes Alkylglucosid (a) mit R1 gleich einer C₂₀-Alkylgruppe ist beispielsweise unter dem Handelsnamen Montanov 202 bekannt.

Effektiv ist die Reduzierung der Autoxidation insbesondere auch dann, wenn das oder die Alkylglucoside (a) der Formel (I) in bestimmten Mengenbereichen eingsetzt werden. Das oder die Alkylglucoside der Formel (I) werden in einer Gesamtmenge von von 0,3 bis 4,5 Gew.-%, bevorzugt von 0,5 bis 3,5 Gew.-%, weiter bevorzugt von 0,7 bis 2,5 Gew.-% und besonders bevorzugt von 0,9 bis 1,5 Gew.-% - jeweils bezogen auf das Gesamtgewicht des anwendungsbereiten Mittels - eingesetzt.

Ein erfindungsgemäßes Mittel ist dadurch gekennzeichnet, dass die Zubereitung (A) ein oder mehrere Alkylglucoside (a) der Formel (I) in einer Gesamtmenge von 0,3 bis 4,5 Gew.-%, bevorzugt von 0,5 bis 3,5 Gew.-%, weiter bevorzugt von 0,7 bis 2,5 Gew.-% und besonders bevorzugt von 0,9 bis 1,5 Gew.-% - bezogen auf das Gesamtgewicht des anwendungsbereiten Mittels - enthält.

Als zweiten wesentlichen Formulierungsbestandteil enthalten die erfindungsgemäßen Zubereitungen (A) mindestens ein weiteres Alkylglucosid (b) der Formel (II) 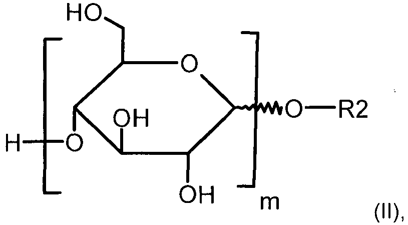 worin R2 für eine unverzweigte oder verzweigte, gesättigte oder ungesättigte C₈-C₁₈-Alkylgruppe steht und m für eine ganze Zahl von 1 bis 10 steht.

Auch die Ausbildung von Glucosiden der Formel (II) erfolgt ausgehend von Glucose in ihrer Pyranoseform, wobei auch hier die Glucoside ausgehend von α-Glucose (Formel IIa) als auch von der β-Glucose (Formel IIb) von der Erfindung mit umfasst sind.

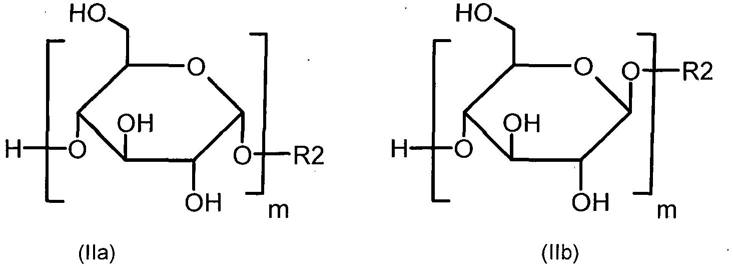

Wenn ausgewählte Vertreter von Alkylglucosiden der Formel (II) zusammen mit den weiteren wesentlichen Bestandteilen (a), (c) und (d) im erfindungsgemäßen Mittel eingesetzt werden, sind die entsprechenden Mittel besonders unempfindlich gegenüber atmosphärischem Sauerstoff und verfärben sich unter seinem Einfluss entsprechend wenig.

Es ist daher bevorzugt wenn der Rest R2 für eine unverzweigte, gesättigte C₁₂-C₁₈-Alkylgruppe steht. Weiter bevorzugt steht der Rest R2 für eine unverzweigte, gesättigte C₁₄-C₁₆-Alkylgruppe, und ganz besonders bevorzugt steht der Rest R2 für eine unverzweigte, gesättigte C₁₆-C₁₈-Alkylgruppe. Weiterhin ist es zur Lösung der Aufgabenstellung vorteilhaft, wenn m für eine ganze Zahl von 1 bis 4 steht. Weiter bevorzugt steht m die Zahlen 1 oder 2, und explizit ganz besonders bevorzugt steht m für die Zahl 1.

Ganz besonders bevorzugt wird als Alkylglucosid der Formel (II) mindestens eine Verbindung ausgewählt aus den Formeln (IIc) und (IId) eingesetzt.

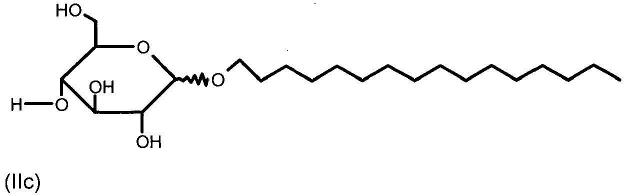

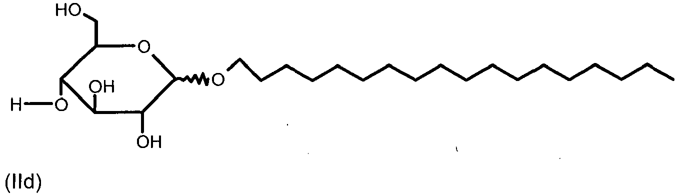

In einer weiteren besonders bevorzugten Ausführungsform ist ein erfindungsgemäßes anwendungsbereites Mittel dadurch gekennzeichnet, dass die Zubereitung (A) als zweites Alkylglucosid (b) mindestens eine Verbindung der Formel (II) enthält, bei welcher
R2 für eine unverzweigte, gesättigte C₁₆-Alkylgruppe oder eine unverzweigte, gesättigte C₁₈-Alkylgruppe steht und
m für die Zahl 1 steht.

Ein besonders bevorzugtes Alkylglucosid (b) mit R2 gleich einer C₁₆-C₁₈-Alkylgruppe ist beispielsweise unter dem Handelsnamen Montanov 68 bekannt.

Im Hinblick auf eine optimale Lösung der erfindungsgemäßen Aufgabenstellung sind auch das bzw. die Alkylglucoside der Formel (II) bevorzugt in bestimmten Mengenbereichen im erfindungsgemäßen Mittel enthalten. Besonders bevorzugt ist es, wenn die erfindungsgemäße Zubereitung (A) ein oder mehrere Alkylglucoside (b) der Formel (II) in einer Gesamtmenge von 1,2 bis 8,0 Gew.-%, bevorzugt von 1,6 bis 6,5 Gew.-%, weiter bevorzugt von 2,0 bis 5,0 Gew.-% und besonders bevorzugt von 2,4 bis 3,5 Gew.-% - bezogen auf das Gesamtgewicht des anwendungsbereiten Mittels - enthält. Daher ist ein erfindungsgemäßes anwendungsbereites Mittel in einer weitere besonders bevorzugte Ausführungsform dadurch gekennzeichnet, dass die Zuberereitung (A) ein oder mehrere Alkylglucoside (b) der Formel (II) in einer Gesamtmenge von 1,2 bis 8,0 Gew.-%, bevorzugt von 1,6 bis 6,5 Gew.-%, weiter bevorzugt von 2,0 bis 5,0 Gew.-% und besonders bevorzugt von 2,4 bis 3,5 Gew.-% - bezogen auf das Gesamtgewicht des anwendungsbereiten Mittels - enthält.

Darüber hinaus hat sich herausgestellt, dass sich die erfindungsgemäßen Färbemittel insbesondere dann in Anwesenheit von Luftsauerstoff wenig farblich verändern, wenn die Alkylglucoside der Formel (I) und die Alkylglucoside der Formel (II) in bestimmten Mengenverhältnissen zueinander eingesetzt werden.

In diesem Zusammenhang ist es insbesondere von Vorteil, wenn die Gesamtmenge der im anwendungsbereiten Mittel eingesetzten Alkylglucoside der Formel (II) mindestens doppelt so hoch ist wie die Gesamtmenge der im anwendungsbereiten Mittel enthaltenen Alkylglucoside der Formel (I).

In einer weiteren ganz besonders bevorzugten Ausführungsform ist ein erfindungsgemäßes Mittel daher dadurch gekennzeichnet, dass das Mengenverhältnis aller im anwendungsbereiten Mittel enthaltenen Alkylglucoside der Formel (I) zu allen im anwendungsbereiten Mittel enthaltenen Alkylglucosiden der Formel (II) bei 1: 2 bis 1:10, bevorzugt bei 1:2 bis 1:5, liegt.

Als dritten wesentlichen Formulierungsbestandteil (c) enthalten die Zubereitungen (A) des erfindungsgemäßen anwendungbereiten Mittels zum Färben und/oder Aufhellen von Keratinfasern mindestens ein Oxidationsfarbstoffvorprodukt vom Entwicklertyp.

Geeignete Oxidationsfarbstoffvorprodukte vom Entwickler-Typ sind dabei ausgewählt aus mindestens einer Verbindung aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxy-ethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Überraschenderweise konnte im Zuge der zu dieser Erfindung führenden Arbeiten gefunden werden, das die speziellen Kombinationen von Alkylglucosiden der Formel (I) und Alkylglucosiden der Formel (II) insbesondere dann die Autoxidation der erfindungsgemäßen Mittel in vorteilhafter Weise minimieren, wenn die Mittel als Oxidationsfarbstoffvorprodukt vom Entwicklertyp p-Toluylendiamin (2,5-Diamino-toluen) enthalten.

In einer weiteren besonders bevorzugten Ausführungsform ist ein erfindungsgemäßes Mittel daher dadurch gekennzeichnet, dass die Zubereitung (A) als Oxidationsfarbstoffvorprodukt vom Entwicklertyp (c) Toluylendiamin (2,5-Diamino-toluene) enthält.

Toluylendiamin (2,5-Diamino-toluene) kann in oxidativen Färbemitteln in Form seiner freien Base, oder in Form eines seiner phyisologischen Salze eingesetzt werden. Physiologisch verträgliche Salze sind beispielsweise
Toluylendiamin (2,5-Diamino-toluene), Hydrochlorid
Toluylendiamin (2,5-Diamino-toluene), Hydrobromid und
Toluylendiamin (2,5-Diamino-toluene), Sulfat
Es hat sich herausgestellt, dass die Autoxidation von erfindungsgemäßen Mitteln, die Toluylendiamin (2,5-Diamino-toluene) in Form des Sulfatsalzes enthalten, besonders wirkungsvoll unterdrückt werden kann, und dass sich entsprechende Formulierungen am wenigsten farblich verändern, wenn sie Luftsauerstoff ausgesetzt sind.

In einer weiteren explizit ganz besonders bevorzugten Ausführungsform ist ein erfindungsgemäßes Mittel daher dadurch gekennzeichnet, dass die Zubereitung (A) Toluylendiamin (2,5-Diamino-toluene) in Form seines Sulfatsalzes (Toluylendiamin (2,5-Diamino-toluene), Sulfate), enthält.

Wirkungsvoll und effektiv unterdrückt werden kann die Autoxidation der erfindungsgemäßen Mittel insbesondere bei bestimmten Einsatzmengen des Sulfatsalzes von p-Toluylendiamin. Das Sulfatsalz von p-Toluylendiamin wird daher in Mengen von 0,45 bis 1,45 Gew.-%, bevorzugt von 0,55 bis 1,35 Gew.-%, weiter bevorzugt von 0,65 bis 1,25 Gew.-% und besonders bevorzugt von 0,70 bis 1,05 Gew.-% - bezogen auf das Gesamtgewicht des anwendungsbereiten Mittels - eingesetzt. Berechnungsgrundlage für die Einsatzmengen von p-Toluylendiamin, Sulfat ist die Verbindung der nachfolgenden Struktur mit einer molaren Masse von 220,25 g/mol.

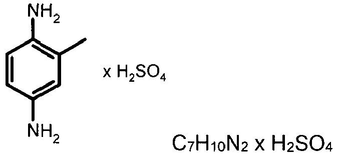

In einer weiteren ganz besonders bevorzugten Ausführungsform ist ein erfindungsgemäßes Mittel dadurch gekennzeichnet, dass die Zubereitung (A) als Oxidationsfarbstoffvorprodukt vom Entwicklertyp (c) p-Toluylendiamin, welches in Form seines Sulfatsalzes vorliegt, in einer Menge von 0,45 bis 1,45 Gew.-%, bevorzugt von 0,55 bis 1,35 Gew.-%, weiter bevorzugt von 0,65 bis 1,25 Gew.-% und besonders bevorzugt von 0,70 bis 1,05 Gew.-% - bezogen auf das Gesamtgewicht des anwendungsbereiten Mittels - enthält.

Als vierten wesentlichen Bestandteil (d) enthalten die erfindungsgemäßen Zubereitungen (A) mindestens ein Oxidationsfarbstoffvorprodukt vom Kupplertyp.

Geeignete Oxidationsfarbstoffvorprodukte vom Kuppler-Typ sind ausgewählt aus der Gruppe 3-Aminophenol, 5-Amino-2-methylphenol, 3-Amino-2-chlor-6-methylphenol, 2-Hydroxy-4-amino phenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Di-hydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Es hat sich herausgestellt, dass die Verfärbung der erfindungsgemäßen Mittel insbesondere dann wirkungsvoll minimiert werden kann, wenn in den erfindungsgemäßen Mitteln ganz spezielle Kombinationen der Bestandteile (a), (b) und (c) mit Oxidationsfarbstoffvorprodukten vom Kupplertyp (d) eingesetzt werden.

Besonders geringe durch Luftsauerstoff hervorgerufene Verfärbungen konnten bei erfindungsgemäßen Mitteln beobachtet werden, die als Oxidatonsfarbstoffvorprodukt Kupplertyp ein oder mehrere Resorcinderivate ausgewählt aus der Gruppe Resorcin, 2-Methylresorcin und 4-Chlorresorcin enthalten.

In einer weitern besonders bevorzugten Ausführungsform ist ein erfindungsgemäßes Mittel deshalb dadurch gekennzeichnet, dass es als Oxidatonsfarbstoffvorprodukt Kupplertyp ein oder mehrere Resorcinderivate ausgewählt aus der Gruppe Resorcin, 2-Methylresorcin und 4- Chlorresorcin enthält.

Von besonderem Vorteil ist es, wenn das oder die Resorcinderivate aus der vorgenannten Gruppe in bestimmten Mengenbereichen im erfindungsgemäßen Mittel enthalten sind.

In einer weiteren besonders bevorzugten Ausführungsform ist eine erfindungsgemäße Zubereitung (A) dadurch gekennzeichnet, dass es als Oxidatonsfarbstoffvorprodukt vom Kupplertyp (d) ein oder mehrere Resorcinderivate ausgewählt aus der Gruppe Resorcin, 2-Methylresorcin und 4-Chlorresorcin in einer Gesamtmenge von 0,10 bis 0,85 Gew.-%, bevorzugt von 0,15 bis 0,75 Gew.-%, weiter bevorzugt von 0,20 bis 0,65 Gew.-% und besonders bevorzugt von 0,25 bis 0,55 Gew.-% - bezogen auf das Gesamtgewicht des anwendungsbereiten Mittels - enthält.

Weiterhin hat sich herausgestellt, dass sich auch die Autoxidation der Nuancen, bei denen m-Aminophenolderivate zur Färbung der Keratinfaser eingesetzt werden, in vorteilhafterweise Weise durch den Einsatz von Kombinationen aus einem erstes Alkylglucosid der Formel (I) und einem zweiten Alkylglucosid der Formel (II) minimieren lassen.

In einer weiteren besonders bevorzugten Ausführungsform enthalten die erfindungsgemäßen Zubereitungen (A) daher als Oxidationsfarbstoffvorprodukt vom Kupplertyp (d) ein oder mehrere m-Aminophenolderivate ausgewählt aus der Gruppe m-Aminophenol, 5-Amino-2-methylphenol und 3-Amino-2-chlor-6-methylphenol.

Von besonderem Vorteil ist es ebenfalls, wenn das oder die m-Aminophenolderivate aus der vorgenannten Gruppe in bestimmten Mengenbereichen im erfindungsgemäßen Mittel enthalten sind. In einer weiteren besonders bevorzugten Ausführungsform ist eine erfindungsgemäße Zubereitung (A) dadurch gekennzeichnet, dass es als Oxidationsfarbstoffvorprodukt vom Kupplertyp (d) ein oder mehrere m-Aminophenolderivate ausgewählt aus der Gruppe m-Aminophenol, 5-Amino-2-methylphenol und 3-Amino-2-chlor-6-methylphenol in einer Gesamtmenge von 0,01 bis 0,55 Gew.-%, bevorzugt von 0,03 bis 0,45 Gew.-%, weiter bevorzugt von 0,05 bis 0,35 und besonders bevorzugt von 0,07 bis 0,25 Gew.-% - bezogen auf das Gesamtgewicht des anwendungsbereiten Mittels - enthält.

Schließlich konnte ebenfalls gefunden werden, dass die Autoxidation der Nuancen, bei denen 2-Aminopyridinderivate zur Färbung der Keratinfaser eingesetzt werden, in vorteilhafterweise Weise durch den Einsatz von Kombinationen aus einem erstes Alkylglucosid der Formel (I) und einem zweiten Alkylglucosid der Formel (II) minimieren lassen.

In einer weiteren besonders bevorzugten Ausführungsform enthalten die erfindungsgemäßen Zubereitungen (A) daher als Oxidationsfarbstoffvorprodukt vom Kupplertyp (d) ein oder mehrere 2-Aminopyridinderivate ausgewählt aus der Gruppe 3-Amino-2-methylamino-6-methoxypyridin und 2-Amino-3-hydroxypyridin.

Von besonderem Vorteil ist es ebenfalls, wenn das oder die 2-Aminopyridinderivate aus der vorgenannten Gruppe in bestimmten Mengenbereichen in den erfindungsgemäßen Zubereitungen (A) enthalten sind. In einer weiteren besonders bevorzugten Ausführungsform ist ein erfindungsgemäßes Mittel dadurch gekennzeichnet, dass es als Oxidationsfarbstoffvorprodukt vom Kupplertyp (d) ein oder mehrere 2-Aminopyridinderivate ausgewählt aus der Gruppe 3-Amino-2-methylamino-6-methoxypyridin und 2-Amino-3-hydroxypyridin in einer Gesamtmenge von 0,001 bis 0,35 Gew.-%, bevorzugt von 0,005 bis 0,25 Gew.-%, weiter bevorzugt von 0,01 bis 0,15 Gew.-% und besonders bevorzugt von 0,02 bis 0,05 Gew.-% - bezogen auf das Gesamtgewicht des anwendungsbereiten Mittels - enthält.

Unter Berücksichtigung der zuvor genannten bevorzugten und besonders bevorzugten Mengenbereiche der erfindungswesentlichen Bestandteile (a), (b) und (c) sowie der besonders bevorzugten Kuppler und Einsatzmengen der Kuppler (d) ist ein besonders bevorzugtes erfindungsgemäßes anwendungsbereites Mittel dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,3 bis 4,5 Gew.-%
(b) ein o. mehrere Alkylglucoside der Formel (II) in einer Gesamtmenge von 1,2 bis 8,0 Gew.-%
(c) als Oxidationsfarbstoffvorprodukt vom Entwicklertyp Toluylendiamin, Sulfat in einer Menge von 0,45 bis 1,45 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,3 bis 4,5 Gew.-%
(b) ein o. mehrere Alkylglucoside der Formel (II) in einer Gesamtmenge von 1,2 bis 8,0 Gew.-%
(c) als Oxidationsfarbstoffvorprodukt vom Entwicklertyp Toluylendiamin, Sulfat in einer Menge von 0,65 bis 1,25 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,3 bis 4,5 Gew.-%
(b) ein o. mehrere Alkylglucoside der Formel (II) in einer Gesamtmenge von 1,2 bis 8,0 Gew.-%
(c) als Oxidationsfarbstoffvorprodukt vom Entwicklertyp Toluylendiamin, Sulfat in einer Menge von 0,70 bis 1,05 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,3 bis 4,5 Gew.-%
(b) ein o. mehrere Alkylglucoside der Formel (II) in einer Gesamtmenge von 1,6 bis 6,5 Gew.-%
(c) als Oxidationsfarbstoffvorprodukt vom Entwicklertyp Toluylendiamin, Sulfat in einer Menge von 0,55 bis 1,35 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,3 bis 4,5 Gew.-%
(b) ein o. mehrere Alkylglucoside der Formel (II) in einer Gesamtmenge von 1,6 bis 6,5 Gew.-%
(c) als Oxidationsfarbstoffvorprodukt vom Entwicklertyp Toluylendiamin, Sulfat in einer Menge von 0,70 bis 1,05 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,3 bis 4,5 Gew.-%
(b) ein o. mehrere Alkylglucoside der Formel (II) in einer Gesamtmenge von 2,0 bis 5,0 Gew.-%
(c) als Oxidationsfarbstoffvorprodukt vom Entwicklertyp Toluylendiamin, Sulfat in einer Menge von 0,45 bis 1,45 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,3 bis 4,5 Gew.-%
(b) ein o. mehrere Alkylglucoside der Formel (II) in einer Gesamtmenge von 2,0 bis 5,0 Gew.-%
(c) als Oxidationsfarbstoffvorprodukt vom Entwicklertyp Toluylendiamin, Sulfat in einer Menge von 0,65 bis 1,25 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,3 bis 4,5 Gew.-%
(b) ein o. mehrere Alkylglucoside der Formel (II) in einer Gesamtmenge von 2,4 bis 3,5 Gew.-%
(c) als Oxidationsfarbstoffvorprodukt vom Entwicklertyp Toluylendiamin, Sulfate in einer Menge von 0,45 bis 1,45 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,3 bis 4,5 Gew.-%
(b) ein o. mehrere Alkylglucoside der Formel (II) in einer Gesamtmenge von 2,4 bis 3,5 Gew.-%
(c) als Oxidationsfarbstoffvorprodukt vom Entwicklertyp Toluylendiamin, Sulfate in einer Menge von 0,55 bis 1,35 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,3 bis 4,5 Gew.-%
(b) ein o. mehrere Alkylglucoside der Formel (II) in einer Gesamtmenge von 2,4 bis 3,5 Gew.-%
(c) als Oxidationsfarbstoffvorprodukt vom Entwicklertyp Toluylendiamin, Sulfate in einer Menge von 0,65 bis 1,25 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,5 bis 3,5 Gew.-%
(b) ein o. mehrere Alkylglucoside der Formel (II) ein einer Gesamtmenge von 1,2 bis 8,0 Gew.-%
(c) als Oxidationsfarbstoffvorprodukt vom Entwicklertyp Toluylendiamin, Sulfate in einer Menge von 0,45 bis 1,45 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,5 bis 3,5 Gew.-%
(b) ein o. mehrere Alkylglucoside der Formel (II) ein einer Gesamtmenge von 1,2 bis 8,0 Gew.-%
(c) als Oxidationsfarbstoffvorprodukt vom Entwicklertyp Toluylendiamin, Sulfate in einer Menge von 0,55 bis 1,35 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,5 bis 3,5 Gew.-%
(b) ein o. mehrere Alkylglucoside der Formel (II) ein einer Gesamtmenge von 1,2 bis 8,0 Gew.-%
(c) als Oxidationsfarbstoffvorprodukt vom Entwicklertyp Toluylendiamin, Sulfate in einer Menge von 0,65 bis 1,25 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,5 bis 3,5 Gew.-%
(b) ein o.mehrere Alkylglucoside der Formel (II) ein einer Gesamtmenge von 1,6 bis 6,5 Gew.-%
(c) als Oxidationsfarbstoffvorprodukt vom Entwicklertyp Toluylendiamin, Sulfate in einer Menge von 0,45 bis 1,45 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,5 bis 3,5 Gew.-%
(b) ein o.mehrere Alkylglucoside der Formel (II) ein einer Gesamtmenge von 1,6 bis 6,5 Gew.-%
(c) als Oxidationsfarbstoffvorprodukt vom Entwicklertyp Toluylendiamin, Sulfate in einer Menge von 0,55 bis 1,35 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,5 bis 3,5 Gew.-%
(b) ein o.mehrere Alkylglucoside der Formel (II) ein einer Gesamtmenge von 1,6 bis 6,5 Gew.-%
(c) als Oxidationsfarbstoffvorprodukt vom Entwicklertyp Toluylendiamin, Sulfate in einer Menge von 0,65 bis 1,25 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,5 bis 3,5 Gew.-%
(b) ein o. mehrere Alkylglucoside der Formel (II) in einer Gesamtmenge von 2,0 bis 5,0 Gew.-%
(c) als Oxidationsfarbstoffvorprodukt vom Entwicklertyp Toluylendiamin, Sulfate in einer Menge von 0,45 bis 1,45 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,5 bis 3,5 Gew.-%
(b) ein o. mehrere Alkylglucoside der Formel (II) in einer Gesamtmenge von 2,0 bis 5,0 Gew.-%
(c) als Oxidationsfarbstoffvorprodukt vom Entwicklertyp Toluylendiamin, Sulfate in einer Menge von 0,65 bis 1,25 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,5 bis 3,5 Gew.-%
(b) ein o. mehrere Alkylglucoside der Formel (II) in einer Gesamtmenge von 2,0 bis 5,0 Gew.-%
(c) als Oxidationsfarbstoffvorprodukt vom Entwicklertyp Toluylendiamin, Sulfate in einer Menge von 0,70 bis 1,05 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,5 bis 3,5 Gew.%
(b) ein o. mehrere Alkylglucoside der Formel (II) ein einer Gesamtmenge von 2,4 bis 3,5 Gew.-%
(c) als Oxidationsfarbstoffvorprodukt vom Entwicklertyp Toluylendiamin, Sulfate in einer Menge von 0,45 bis 1,45 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,5 bis 3,5 Gew.-%
(b) ein o. mehrere Alkylglucoside der Formel (II) ein einer Gesamtmenge von 2,4 bis 3,5 Gew.-%
(c) als Oxidationsfarbstoffvorprodukt vom Entwicklertyp Toluylendiamin, Sulfate in einer Menge von 0,65 bis 1,25 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,5 bis 3,5 Gew.-%
(b) ein o. mehrere Alkylglucoside der Formel (II) ein einer Gesamtmenge von 2,4 bis 3,5 Gew.-%
(c) als Oxidationsfarbstoffvorprodukt vom Entwicklertyp Toluylendiamin, Sulfate in einer Menge von 0,70 bis 1,05 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,7 bis 2,5 Gew.-%
(b) ein o. mehrere Alkylglucoside der Formel (II) in einer Gesamtmenge von 1,2 bis 8,0 Gew.-%
(c) als Oxidationsfarbstoffvorprodukt vom Entwicklertyp Toluylendiamin, Sulfate in einer Menge von 0,55 bis 1,35 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,7 bis 2,5 Gew.-%
(b) ein o. mehrere Alkylglucoside der Formel (II) in einer Gesamtmenge von 1,2 bis 8,0 Gew.-%
(c) als Oxidationsfarbstoffvorprodukt vom Entwicklertyp Toluylendiamin, Sulfate in einer Menge von 0,65 bis 1,25 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,7 bis 2,5 Gew.-%
(b) ein o. mehrere Alkylglucoside der Formel (II) in einer Gesamtmenge von 1,2 bis 8,0 Gew.-%
(c) als Oxidationsfarbstoffvorprodukt vom Entwicklertyp Toluylendiamin, Sulfate in einer Menge von 0,70 bis 1,05 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,7 bis 2,5 Gew.-%
(b) ein o. mehrere Alkylglucoside der Formel (II) in einer Gesamtmenge von 1,6 bis 6,5 Gew.-%
(c) als Oxidationsfarbstoffvorprodukt vom Entwicklertyp Toluylendiamin, Sulfate in einer Menge von 0,55 bis 1,35 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,7 bis 2,5 Gew.-%
(b) ein o. mehrere Alkylglucoside der Formel (II) in einer Gesamtmenge von 1,6 bis 6,5 Gew.-%
(c) als Oxidationsfarbstoffvorprodukt vom Entwicklertyp Toluylendiamin, Sulfate in einer Menge von 0,65 bis 1,25 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,7 bis 2,5 Gew.-%
(b) ein o. mehrere Alkylglucoside der Formel (II) in einer Gesamtmenge von 2,0 bis 5,0 Gew.-%
(c) als Oxidationsfarbstoffvorprodukt vom Entwicklertyp Toluylendiamin, Sulfate in einer Menge von 0,45 bis 1,45 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,7 bis 2,5 Gew.-%
(b) ein o. mehrere Alkylglucoside der Formel (II) in einer Gesamtmenge von 2,0 bis 5,0 Gew.-%
(c) als Oxidationsfarbstoffvorprodukt vom Entwicklertyp Toluylendiamin, Sulfate in einer Menge von 0,55 bis 1,35 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,7 bis 2,5 Gew.-%
(b) ein o. mehrere Alkylglucoside der Formel (II) in einer Gesamtmenge von 2,0 bis 5,0 Gew.-%
(c) als Oxidationsfarbstoffvorprodukt vom Entwicklertyp Toluylendiamin, Sulfate in einer Menge von 0,70 bis 1,05 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,7 bis 2,5 Gew.-%
(b) ein o. mehrere Alkylglucoside der Formel (II) in einer Gesamtmenge von 2,4 bis 3,5 Gew.-%
(c) als Oxidationsfarbstoffvorprodukt vom Entwicklertyp Toluylendiamin, Sulfate in einer Menge von 0,55 bis 1,35 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,7 bis 2,5 Gew.-%
(b) ein o. mehrere Alkylglucoside der Formel (II) in einer Gesamtmenge von 2,4 bis 3,5 Gew.-%
(c) als Oxidationsfarbstoffvorprodukt vom Entwicklertyp Toluylendiamin, Sulfate in einer Menge von 0,70 bis 1,05 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 1,2 bis 8,0 Gew.-%
(c) als Oxidationsfarbstoffvorprodukt vom Entwicklertyp Toluylendiamin, Sulfate in einer Menge von 0,45 bis 1,45 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 1,2 bis 8,0 Gew.-%
(c) als Oxidationsfarbstoffvorprodukt vom Entwicklertyp Toluylendiamin, Sulfate in einer Menge von 0,65 bis 1,25 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 1,6 bis 6,5 Gew.-%
(c) als Oxidationsfarbstoffvorprodukt vom Entwicklertyp Toluylendiamin, Sulfate in einer Menge von 0,55 bis 1,35 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 1,6 bis 6,5 Gew.-%
(c) als Oxidationsfarbstoffvorprodukt vom Entwicklertyp Toluylendiamin, Sulfate in einer Menge von 0,65 bis 1,25 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,0 bis 5,0 Gew.-%
(c) als Oxidationsfarbstoffvorprodukt vom Entwicklertyp Toluylendiamin, Sulfate in einer Menge von 0,45 bis 1,45 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,0 bis 5,0 Gew.-%
(c) als Oxidationsfarbstoffvorprodukt vom Entwicklertyp Toluylendiamin, Sulfate in einer Menge von 0,55 bis 1,35 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,0 bis 5,0 Gew.-%
(c) als Oxidationsfarbstoffvorprodukt vom Entwicklertyp Toluylendiamin, Sulfate in einer Menge von 0,70 bis 1,05 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,4 bis 3,5 Gew.-%
(c) als Oxidationsfarbstoffvorprodukt vom Entwicklertyp Toluylendiamin, Sulfate in einer Menge von 0,45 bis 1,45 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,4 bis 3,5 Gew.-%
(c) als Oxidationsfarbstoffvorprodukt vom Entwicklertyp Toluylendiamin, Sulfate in einer Menge von 0,65 bis 1,25 Gew.-% und
(d) mindestens ein Oxidationsfarbstoffvorprodukt vom Kupplertyp enthält.

Ein weiteres besonders bevorzugtes anwendungsbereites Mittel ist dadurch gekennzeichnet, dass die Zubereitung (A) - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,4 bis 3,5 Gew.-%
(c) als Oxidationsfarbstoffvorprodukt vom Entwicklertyp Toluylendiamin, Sulfate in einer Menge von 0,70 bis 1,05 Gew.-% und
(d) mindestens ein Oxidationsfarbstoffvorprodukt vom Kupplertyp enthält.

Auch wenn bestimmte Kombinationen von Oxidationsfarbstoffvorprodukten vom Kupplertyp (d) in den erfindungsgemäßen Mitteln eingesetzt werden, kann die durch Luftsauerstoff hervorgerufene Autoxidation der Mittel besonders wirkungsvoll minimiert bzw.sogar nahezu verhindert werden. Gute Ergebnisse werden insbesondere erhalten, wenn als Oxidationsfarbstoffe vom Kupplertyp (d) eine Kombination von Resorcin und m-Aminophenol eingesetzt wird.

In einer weiteren besonders bevorzugten Ausführungsform ist ein erfindungsgemäßes anwendungsbereites Mittel daher dadurch gekennzeichnet, dass die Zubereitung (A) als Oxidationsfarbstoffvorprodukte vom Kupplertyp (d) Resorcin und m-Aminophenol enthält.

Auch wenn als Oxidationsfarbstoffe vom Kupplertyp (d) eine Kombination aus Resorcin und 3-Amino-2-methylamino-6-methoxypyridin eingesetzt werden, sind die durch Luftsauerstoff hervorgerufenen farblichen Veränderungen der Mittel besonders gering.

In einer weiteren besonders bevorzugten Ausführungsform ist ein erfindungsgemäßes anwendungsbereites Mittel daher dadurch gekennzeichnet, dass die Zubereitung (A) als Oxidationsfarbstoffvorprodukte vom Kupplertyp (d) Resorcin und 3-Amino-2-methylamino-6-methoxypyridin enthält. Weiterhin hat es sich im Hinblick auf die Minimierung der Autoxidation als besonders vorteilhaft herausgestellt, wenn die erfindungsgemäßen Mittel als Oxidationsfarbstoffvorprodukte vom Kupplertpyp (d) eine Kombination aus m-Aminophenol und 3-Amino-2-methylamino-6-methoxypyridin enthalten.

In einer weiteren besonders bevorzugten Ausführungsform ist ein erfindungsgemäßes anwendungsbereites Mittel daher dadurch gekennzeichnet, dass die Zubereitung (A) als Oxidationsfarbstoffvorprodukte vom Kupplertyp (d) m-Aminophenol und 3-Amino-2-methylamino-6-methoxypyridin enthält.

Werden als Oxidationsfarbstoffvorprodukte vom Kupplertyp (d) Resorcin zusammen mit m-Aminophenol und 3-Amino-2-methylamino-6-methoxypyridin eingesetzt, so kann die durch atmosphärischen Luftsauerstoff hervorgerufene Verfärbung der Mittel ebenfalls besonders gut im Zusammenspiel mit den weiteren wesentlichen Inhaltsstoffen (a), (b) und (c) unterdrückt werden. In einer weiteren besonders bevorzugten Ausführungsform ist ein erfindungsgemäßes anwendungsbereites Mittel daher dadurch gekennzeichnet, dass die Zubereitung (A) als Oxidationsfarbstoffvorprodukte vom Kupplertyp (d) Resorcin, m-Aminophenol und 3-Amino-2-methylamino-6-methoxypyridin enthält.

Im Zuge der zu dieser Erfindung führenden Arbeiten hat sich herausgestellt, dass das Ausmaß, in dem die Autoxidation der Oxidationsfarbstoffvorprodukte in den erfindungsgemäßen Mitteln verringert bzw. inhibiert werden kann, auf einem Zusammenspiel der wesentlichen Bestandteile (a) und (b) auf der einen Seite und den wesentlichen Bestandteilen (c) und (d) auf der anderen Seite beruht. Optimal kann die Autoxidation demzufolge inhibiert werden, wenn die Gesamtmenge der im anwendungsbereiten Mittel enthaltenen Alkylgucoside (a) und (b) sich in optimalem Verhältnis zu den in der Gesamtmenge der im anwendungsbereiten Mittel befindlichen Oxditationsfarbstoffvorprodukte (c) und (d) befindet.

Es konnte gefunden werden, dass die effektive Verhinderung der Autoxidation insbesondere dann beobachtet werden kann, wenn die Gesamtmenge der im Mittel enthaltenen Alkylglucoside (a) und (b) mindestens genauso groß ist wie die Gesamtmenge aller im anwendungsbereiten Mittel enthaltenen Oxidationsfarbstoffvorprodukte (c) und (d). Bevorzugt ist die Gesamtmenge der im Mittel enthaltenen Alkylclucoside (a) und (b) mindestens doppelt so groß wie die Gesamtmenge aller im anwendungsbereiten Mittel enthaltenen Oxidationsfarbstoffvorprodukte (c) und (d). Insbesondere bevorzugt ist die Gesamtmenge der im Mittel enthaltenen Alkylglucoside (a) und (b) mindestens dreimal so groß wie die Gesamtmenge aller im anwendungsbereiten Mittel enthaltenen Oxidationsfarbstoffvorprodukte (c) und (d).ln einer weiteren ganz besonders bevorzugten Ausführungsform ist ein erfindungsgemäßes Mittel daher dadurch gekennzeichnet, dass das Mengenverhältnis aller im anwendungsbereiten Mittel enthaltenen Oxidationsfarbstoffvorprodukte zu allen im anwendungsbereiten Mittel enthaltenen Alkylglucosiden bei mindestens 1:1, bevorzugt bei mindestens 1:2 und besonders bevorzugt bei mindestens 1:3 liegt.

Die anwendungsbereiten Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Erfindungsgemäß bevorzugte Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß bevorzugte Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind beispielsweise Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus Gruppe der Amidoamine stellt das unter der Bezeichnung Tegoamid® S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten.

Die anwendungsbereiten Färbemittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind anionische, synthetische Polymere; kationische, synthetische Polymere; natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen; nichtionische, synthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Auch zwitterionische Polymere können in den erfindungsgemäßen Mitteln enthalten sein. Bevorzugte zwitterionische Polymere werden ausgewählt aus der Gruppe der
- Copolymere aus Dimethyl-diallylammoniumsalzen und Acrylsäure, z.B. Polyquaternium-22,
- Copolymere aus Dimethyl-diallylammoniumsalzen und Methacrylsäure,
- Copolymere aus N,N,N-Trimethyl-3-[(2-methyl-1-oxo-2-propen-1-yl)amino]-1-propanaminium-salzen und Acrylsäure,
- Copolymere aus N,N,N-Trimethyl-3-[(2-methyl-1-oxo-2-propen-1-yl)amino]-1-propanaminium-salzen und Methacrylsäure,
- Copolymere aus N,N,N-Trimethyl-2-[(2-methyl-1-oxo-2-propen-1-yl)amino]-1-ethanaminiumsalzen und Acrylsäure,
- Copolymere aus N,N,N-Trimethyl-2-[(2-methyl-1-oxo-2-propen-1-yl)amino]-1-ethanaminiumsalzen und Methacrylsäure,
- Copolymere aus N,N,N-Trimethyl-3-[(2-methyl-1-oxo-2-propen-1-yl)amino]-1-propanaminium-salzen, Acrylsäure und Acrylamid, z.B. Polyquaternium-53
- Copolymere aus N,N,N-Trimethyl-3-[(2-methyl-1-oxo-2-propen-1-yl)amino]-1-propanaminium-salzen, Methacrylsäure und Acrylamid,
- Copolymere aus 1-Ethenyl-3-methyl-1H-imidazoliumsalzen, 1-Ethenyl-1H-imidazol, 1-Ethenyl-2-pyrrolidinon und Methacrylsäure, z.B. Polyquaternium-86,
- Copolymere aus 1-Ethenyl-3-methyl-1H-imidazoliumsalzen, 1-Ethenyl-1H-imidazol, 1-Ethenyl-2-pyrrolidinon und Acrylsäure.

Auch Gemische der vorgenannten bevorzugten zwitterionischen Polymere (c) können in den erfindungsgemäßen Mittel enthalten sein.

Es hat sich weiterhin gezeigt, dass die erfindungsgemäße Aufgabenstellung insbesondere dann vollständig und in zufriedenstellender Weise gelöst werden kann, wenn die erfindungsgemäßen Mittel weitere ausgewählte Formulierungsbestandteile enthalten.

So hat sich herausgestellt, dass die zusätzliche Anwesenheit von bestimmten, höherkettigen Fettalkoholen das farbliche Ergebnis der erfindungsgemäßen Zusammensetzungen noch weiter verbessert. Daher ist es bevorzugt, wenn die erfindungsgemäßen Mittel zusätzlich einen oder mehrere Fettalkohole aus der Gruppe Arachylalkohol (Eisocan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) enthalten.

Besonders geeignete Mittel enthalten einen oder mehrere höherkettige Alkohole der vorgenannten Gruppe in einer Gesamtmenge von 1,0 bis 10,0 Gew.-%, bevorzugt von 1,4 bis 8,0 Gew.-%, weiter bevorzugt von 1,8 bis 6,0 Gew.-% und besonders bevorzugt von 2,0 bis 4,0 Gew.-% - bezogen auf das Gesamtgewicht des anwendungsbereiten Mittels.

In einer weiteren bevorzugten Ausführungsform ist ein erfindungsgemäßes Mittel daher dadurch gekennzeichnet, dass es zusätzlich einen oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) in einer Gesamtmenge von 0,1 bis 10,0 Gew.-%, bevorzugt von 1,4 bis 8,0 Gew.-%, weiter bevorzugt von 1,8 bis 6,0 Gew.-% und besonders bevorzugt von 2,0 bis 4,0 Gew.-% - bezogen auf das Gesamtgewicht des anwendungsbereiten Mittels - enthält.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 6 und 11, insbesondere zwischen 7 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Werteserfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-) Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin.

Es hat sich als vorteilhaft erwiesen, wenn die erfindungsgemäßen Oxidationsmittelzubereitungen zur Stabilisierung des Wasserstoffperoxids zusätzlich mindestens einen Stabilisator oder Komplexbildner enthalten. Besonders bevorzugte Stabilisatoren sind insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Zusätzlich zu den erfindungswesentlichen Inhaltsstoffen (a), (b) und (c) können die erfindungsgemäßen Mittel auch Reduktionsmittel enthalten. Geeignete Reduktionsmittel sind Sulfite wir Natriumsulft, Kaliumsulfit, Natriumbisulfit oder Kaliumbisulfit. Auch Ascorindsäure, Isoascorbinsäure oder Dehydoascorbinsäure sind in diesem Zusammenhang geeignet.

Obwohl es im Hinblick auf die Verhinderung der Autoxidation der Oxidationsfarbstoffvorprodukte von Vorteil ist, den erfindungsgemäßen Mitteln Reduktionsmittel zuzusetzen, so ist deren Einsatz auch mit anwendungstechnischen Nachteilen verbunden. Werden Reduktionsmittel in zu hohen Mengen eingesetzt können, kann sich diese beispielsweise negativ auf die Farbleistung und Farbintensität auswirken.

Die Gesamtmenge der eingesetzten Reduktionsmittel liegt deshalb nach Möglichkeit unterhalb von 0,5 Gew.-%, bevorzugt unterhalb von 0,3 Gew.-%, und besonders bevorzugt unterhalb von 0,2 Gew.-% - bezogen auf die Gesamtmenge des anwendungsbereiten Mittels.

In einer weiteren bevorzugten Ausführungsform ist ein erfindungsgemäßes Mittel demzufolge dadurch gekennzeichnet, dass es Reduktionsmittel, ausgewählt aus der Gruppe Natriumsulfit, Natriumbisulfit, Kaliumsulfit, Kaliumbisulfit, Ascorbinsäure und Isoascorbinsäure, in einer Gesamtmenge von weniger als 0,5 Gew.-%, bevorzugt von weniger als 0,3 Gew.-% und besonders bevorzugt von weniger als 0,2 Gew.-% - bezogen auf das Gesamtgewicht des anwendungsbereiten Mittels - enthält.

Zusätzlich zu den vorgenannten Inhaltsstoffen können die erfindungsgemäßen Mittel weiterhin die für oxidative Färbe- und Aufhellmittel üblichen Inhaltsstoffe enthalten. Zusätzlich zu den Oxidationsfarbstoffvorprodukten können die Mittel daher auch direktziehende Farbstoffe enthalten, wobei diese direktziehenden Farbstoffe ausgewählt sein können aus den kationischen, anionischen und nichtionischen Farbstoffen.

Bei den erfindungsgemäßen Mitteln handelt es sich um Mittel zum oxidativen Färben und/oder Aufhellen von Haaren. Im anwendungsbereiten Mittel reagieren die Oxidationsfarbstoffvorprodukte mit dem Oxidationsmittel unter Ausbildung der eigentlichen Farbstoffe. Üblicherweise werden die erfindungsgemäßen Mittel daher als Mehrkomponentenmittel, meist als Zweikomponenten-Mittel, konfektioniert. Die erste Komponente (A) beinhaltet hierbei die Oxidationsfarbstoffvorprodukte (c) und (d), welche kurz vor der Anwendung mit einer zweiten Komponente enthaltend das Oxidationsmittel (Zubereitung B) vermischt wird. Üblicherweise werden beide Komponenten im Verhältnis 1:3 bis 3:1 miteinander vermischt. Bei dieser Mischung der Komponente enthaltend Farbcreme und gegebenenfalls Alkalisierungsmittel (Zubereitung A) und der Komponente enthaltend Oxidationsmittel (Zubereitung B) spricht man von der Anwendungsmischung oder dem anwendungsbereiten Mittel. In einer bevorzugten Ausführungsform sind die Alkylgucoside der Formeln (I) und (II) ebenfalls in der ersten Komponente (Zubereitung A) enthalten. Alle Mengenangaben mit Bezugnahme auf das "anwendungsbereite Mittel" beziehen sich auf die anwendungsbereite Mischung aus der Komponente enthaltend Farbcreme/Alkalisierungsmittel und der Komponente enthaltend Oxidationsmittel.

Ein anwendungsbereites Mittel zum Färben und/oder Aufhellen von keratinischen Fasern ist dadurch gekennzeichnet, dass es unmittelbar vor der Anwendung durch Vermischen der Zubereitungen (A) und (B) hergestellt wird, wobei
- das durch Vermischen der Zubereitungen (A) und (B) hergestellte anwendungsbereite Mittel Wasserstoffperoxid in einer Menge von 0,5 bis 8,0 Gew.-%, bevorzugt 1,5 bis 6,5 Gew.-%, weiter bevorzugt 2,2 bis 4,5 Gew.-% und besonders bevorzugt 3,2 bis 3,8 Gew.-% (berechnet als 100 %-iges Wasserstoffperoxid) - bezogen auf das Gesamtgewicht des anwendungsbereiten Mittels - enthält.

Die im erfindungsgemäßen Mittel enthaltenen Alkylglucoside der Formel (I) und Alkylglucoside der Formel (II) eignen sich hervorragend zur Verhinderung der Autoxidation von oxidativen Mitteln zum Färben und/oder Aufhellen von keratinischen Fasern, welche - in bevorzugter Ausführungsform spezielle - Oxidationsfarbstoffe vom Entwicklertyp und vom Kupplertyp enthalten.

Ein weiterer Gegenstand der vorliegenden Erfindung ist die Verwendung der Kombination aus
(a) mindestens einem ersten Alkylglucosid der Formel (I) 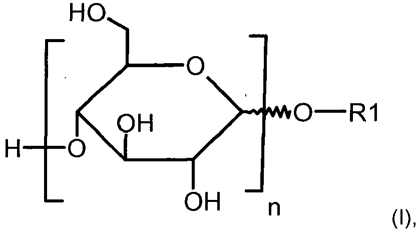
   worin R1 für eine unverzweigte oder verzweigte, gesättigte oder ungesättigte C₂₀-C₂₈-Alkylgruppe steht und
   n für eine ganze Zahl von 1 bis 10 steht und
(b) mindestens einem zweiten Alkylglucosid der Formel (II) 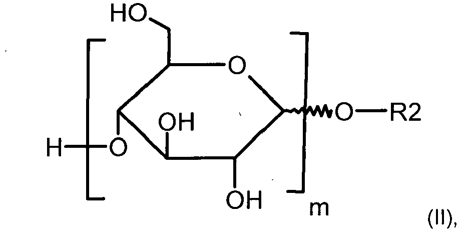
   worin R2 für eine unverzweigte oder verzweigte, gesättigte oder ungesättigte C₈-C₁₈-Alkylgruppe steht und
   m für eine ganze Zahl von 1 bis 10 steht,
   zur Verhinderung der durch atmosphärischen Sauerstoff hervorgerufenen Verfärbung von Mitteln zum oxidativen Färben und/oder Aufhellen von keratinischen Fasern, welche in einem kosmetischen Träger
(c) mindestens ein Oxidationsfarbstoffvorprodukt vom Entwicklertyp und
(d) mindestens ein Oxidationsfarbstoffvorprodukt vom Kupplertyp enthalten.

Unter atmosphärischem Sauerstoff wird der Sauerstoff (O₂) verstanden, der sich in Gasform in der Atmosphäre (Luft, Gasgemisch der Erdatmosphäre) befindet.

Bezüglich weiterer bevorzugter Ausführungsformen der erfindungsgemäßen Verfahren und Verwendungen gilt mutatis mutandis das zu den erfindungsgemäßen Mitteln Gesagte.

### Beispiele

### 1. Herstellung der anwendunqsbereiten Färbemittel

Es wurden die folgenden Rezepturen hergestellt. Die Mengenangaben verstehen sich, sofern nichts anderes vermerkt ist, jeweils in Gewichtsprozent.

| Formulierungsbestandteile | V1 (Gew.-%) | E1 (Gew.-%) |
|---|---|---|
| Montanov 68 (Cetearyl Alcohol, Cetearyl Glucoside) | --- | 6,00 |
| Montanov 202 (Arachidyl Alcohol, Behenyl Alcohol, Arachidyl Glucoside) | --- | 2,00 |
| Paraffinium Liquidum | 7,40 | 7,40 |
| Lanette 22 (INCI: Behenylalcohol) | 1,80 | 1,80 |
| Lanette D (INCI: Cetearylalkohol) | 1,30 | 1,30 |
| Eumulgin B 3 (INCI: Ceteareth-30) | 1,30 | 1,30 |
| Produkt W 37194 (N,N,N-Trimethyl-3-[(1-oxo-2-propenyl)amino]-1-propanaminiumchloride, polymer with sodium 2-propenoate) (INCI: Acrylamidopropyltrimonium chloride /Acrylate Copolymer) | 2,00 | 2,00 |
| p-Toluylendiamin Sulfat | 1,50 | 1,50 |
| Resorcin | 0,58 | 0,58 |
| m-Aminophenol | 0,16 | 0,16 |
| 3-Amino-2-methylamino-6-methoxypyridin | 0,05 | 0,05 |
| Kaliumhydroxid (50 %ig) | 0,7 | 0,7 |
| Vitamin C | 0,05 | 0,05 |
| Hydroxyethan-1,1-diphosphonsäure 60 %ig | 0,20 | 0,20 |
| Natriumsilikat 42 (3,1 SiO₂ : Na₂O) | 0,5 | 0,5 |
| Ammoniak (25 Gew.-%ige wässrige Lösung) | 5,80 | 5,80 |
| Parfum | 0,40 | 0,40 |
| Wasser | ad 100 | ad 100 |

Bei V1 handelt es sich um eine Vergleichsrezeptur, E1 ist eine erfindungsgemäße Formulierung. Die Farbcremes wurden jeweils im Verhältnis 1:1 mit der folgenden Oxidationsmittelformierung (OX) vermischt.

| Formulierungsbestandteile | ox (Gew.-%) |
|---|---|
| Phosphorsäure 85 %ig | 0,04 |
| Wasserstoffperoxid (50 %ige, wässrige Lösung) | 12,00 |
| Emulgade F (INCI: Cetearylalcohol, PEG-40 Castor Oil, Sodium Cetearyl sulfate) | 2,10 |
| Natriumbenzoat | 0,04 |
| Dinatriumpyrophosphat | 0,30 |
| Ethylendiamintetraacetat, Dinatriumsalz | 0,15 |
| Wasser | ad 100 |

### 2. Messung der Autoxidation der Farbcremes

Jeweils 25 g der zuvor hergestellten Farbcremes (V1, E1) wurden auf Petrischalen ausgestrichen und für einen Zeitraum von 60 Minuten an der Luft stehen gelassen. Die Färbung jeder Anwendungsmischung wurde visuell nach einem Zeitraum von 0 Minuten (direkt nach dem Ausstreichen), nach 10 min, 20 min, 30 min, 40 min und 60 beurteilt.

**Tabelle 1: Färbung der Farbcremes**

| Zeitraum [min.] | V1 Färbung (Farbintensität) | E1 Färbung (Farbintensität) |
|---|---|---|
| 0 | beige (-) | ungefärbt (-) |
| 10 | dunkel beige (+) | ungefärbt (-) |
| 20 | hellbraun (++) | beige (+) |
| 30 | mittelbraun (++) | beige-braun (+) |
| 40 | mittelbraun (++) | hellbraun (+) |
| 60 | mittelbraun (++) | hellbraun (+) |

| | | |
|---|---|---|
| Farbintensität: + = niedrig ++ = mittel +++ = hoch | | |

Über den gesamten beobachteten Zeitraum war das erfindungsgemäße Mittel (E1) weniger stark gefärbt als die Vergleichsformulierung (V1).

### 3. Messung der Autoxidation der Anwendungsmischungen

Jeweils 25 g der zuvor zuvor hergestellten Anwendungsmischungen (V1 + OX, E1 + OX) wurden auf Petrischalen ausgestrichen und für einen Zeitraum von 60 Minuten an der Luft stehen gelassen. Die Färbung jeder Anwendungsmischung wurde visuell nach einem Zeitraum von 0 Minuten (direkt nach dem Ausstreichen), nach 10 min, 20 min, 30 min, 40 min und 60 beurteilt.

**Tabelle 2: Färbung der Anwendungsmischung**

| Zeitraum [min.] | V1 + OX Färbung (Farbintensität) | E1 + OX Färbung (Farbintensität) |
|---|---|---|
| 0 | hellbraun (+) | hellbeige (+) |
| 10 | mittelbraun (++) | beige (+) |
| 20 | mittelbraun (++) | mittelbeige (+) |
| 30 | dunkelbraun (+++) | beige-braun (++) |
| 40 | dunkelbraun (+++) | beige-braun (++) |
| 60 | tief dunkelbraun (+++) | braun (++) |

| | | |
|---|---|---|
| Farbintensität: + = niedrig ++ = mittel +++ = hoch | | |

Über den gesamten beobachteten Zeitraum war das erfindungsgemäße Mittel (E1 + OX) weniger stark gefärbt als die Vergleichsformulierung (V1 + OX).

### 4. Ausfärbung und Bestimmung der Farbintensität

Die auf zuvor hergestellten Anwendungmischungen wurden mit einer Aplicette auf Haarsträhnen (Büffelbauchhaar und Haar der Firma Kerling, naturweiß) aufgetragen und dort für einen Zeitraum von 10 bis 30 Minuten belassen. Anschließend wurde die Anwendungsmischung mit einem Schampoo ausgespült und getrocknet. Dann wurden die Haarsträhnen farbmetrisch vermessen (Messung der Lab-wert)

Bei der farbmetrischen Vermessung gibt der L-Wert die Helligkeit einer Strähne wieder, wobei sich der Wertebereich für L von 0 bis 100 erstreckt. Je geringer der L-Wert ist, desto dunkler ist die vermessene Haarsträhne (L = 0 bedeutet schwarz, L = 100 bedeutet (diffuses) weiß).

Es wurden die folgenden L-Werte gemessen:

**Tabelle 3: L-Werte, Büffelbauchhaar**

| | 10 min Anwendungsdauer | 20 min Anwendungsdauer | 30 min Anwendungsdauer |
|---|---|---|---|
| V1 + OX | 26,78 | 18,98 | 16,35 |
| E1 + OX | 23,96 | 17,14 | 15,17 |

**Tabelle 4: L-Werte, Kerling naturweiß**

| | | | |
|---|---|---|---|
| | 10 min Anwendungsdauer | 20 min Anwendungsdauer | 30 min Anwendungsdauer |
| V1 + OX | 29,29 | 22,39 | 20,29 |
| E1 + OX | 27,03 | 17,85 | 17,49 |

Die L-Werte der mit dem erfindungsgemäßen Mittel (E1 + OX) erhaltenen Färbungen waren - unabhängig von der Applikationsdauer - durchweg geringer als jene, die bei Anwendung der Verlgeichsformulierung (V1 + OX) gemessen wurden. Dies bedeutet, dass die mit dem erfindungsgemäßen Mittel gefärbten Haare in einem dunkleren bzw. intensiveren Farbton gefärbt wurden.

Die Verfärbungen der erfindungsgemäßen Mittel selbst (Verfärbung der Formulierungen) waren geringer, trotzdem konnten mit den erfindungsgemäßen Mitteln jedoch intensivere Färbungen auf den Haaren erzielt werden.

## Patentansprüche

1. Anwendungsbereites Mittel zum Färben und/oder Aufhellen von keratinischen Fasern, **dadurch gekennzeichnet, dass** es unmittelbar vor der Anwendung durch Vermischen der Zubereitungen (A) und (B) hergestellt wird, wobei
- es sich bei der Zubereitung (A) um ein Mittel handelt, enthaltend in einem kosmetischen Träger
(a) ein oder mehrere Alkylglucoside der Formel (I) in einer Gesamtmenge von 0,3 bis 4,5 Gew.-% - bezogen auf das Gesamtgewicht des anwendungsbereiten Mittels - 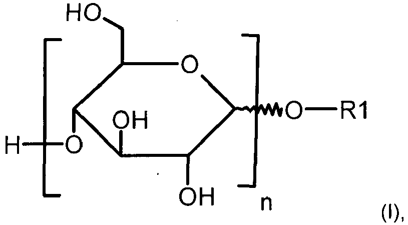
worin R1 für eine unverzweigte oder verzweigte, gesättigte oder ungesättigte C₂₀-C₂₈-Alkylgruppe steht und
n für eine ganze Zahl von 1 bis 10 steht,
(b) mindestens ein zweites Alkylglucosid der Formel (II) 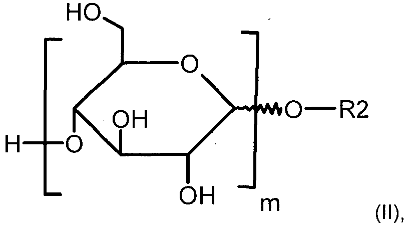
worin R2 für eine unverzweigte oder verzweigte, gesättigte oder ungesättigte C₈-C₁₈-Alkylgruppe steht und
m für eine ganze Zahl von 1 bis 10 steht,
(c) mindestens ein Oxidationsfarbstoffvorprodukt vom Entwicklertyp und
(d) mindestens ein Oxidationsfarbstoffvorprodukt vom Kupplertyp, und
- es sich bei der Zubereitung (B) um ein Mittel handelt, das in einem kosmetischen Träger Wasserstoffperoxid enthält, und
- das durch Vermischen der Zubereitungen (A) und (B) hergestellte anwendungsbereite Mittel Wasserstoffperoxid in einer Menge von 0,5 bis 8,0 Gew.-% (berechnet als 100 %-iges Wasserstoffperoxid) - bezogen auf das Gesamtgewicht des anwendungsbereiten Mittels - enthält.

2. Anwendungsbereites Mittel nach Anspruch 1, **dadurch gekennzeichnet, dass** die Zubereitung (A) ein oder mehrere Alkylglucoside (a) der Formel (I) in einer Gesamtmenge von 0,5 bis 3,5 Gew.-%, bevorzugt von 0,7 bis 2,5 Gew.-% und besonders bevorzugt von 0,9 bis 1,5 Gew.-% - bezogen auf das Gesamtgewicht des anwendungsbereiten Mittels - enthält.

3. Anwendungsbereites Mittel nach einem der Ansprüche 1 oder 2, **dadurch gekennzeichnet, dass** die Zubereitung (A) ein oder mehrere Alkylglucoside (b) der Formel (II) in einer Gesamtmenge von 1,2 bis 8,0 Gew.-%, bevorzugt von 1,6 bis 6,5 Gew.-%, weiter bevorzugt von 2,0 bis 5,0 Gew.-% und besonders bevorzugt von 2,4 bis 3,5 Gew.-% - bezogen auf das Gesamtgewicht des anwendungsbereiten Mittels - enthält.

4. Anwendungsbereites Mittel nach einem der Ansprüche 1 bis 3, **dadurch gekennzeichnet, dass** das Mengenverhältnis aller im anwendungsbereiten Mittel enthaltenen Alkylglucoside der Formel (I) zu allen im anwendungsbereiten Mittel enthaltenen Alkylglucosiden der Formel (II) bei 1: 2 bis 1:10, bevorzugt bei 1:2 bis 1:5, liegt.

5. Anwendungsbereites Mittel nach einem der Ansprüche 1 bis 4, **dadurch gekennzeichnet, dass** die Zubereitung (A) als Oxidationsfarbstoffvorprodukt vom Entwicklertyp (c) p-Toluylendiamin, welches in Form seines Sulfatsalzes vorliegt, in einer Menge von 0,45 bis 1,45 Gew.-%, bevorzugt von 0,55 bis 1,35 Gew.-%, weiter bevorzugt von 0,65 bis 1,25 Gew.-% und besonders bevorzugt von 0,70 bis 1,05 Gew.-% - bezogen auf das Gesamtgewicht des anwendungsbereiten Mittels - enthält.

6. Anwendungsbereites Mittel nach einem der Ansprüche 1 bis 5, **dadurch gekennzeichnet dass** die Zubereitung (A) als Oxidationsfarbstoffvorprodukt vom Kupplertyp (d) ein oder mehrere Resorcinderivate ausgewählt aus der Gruppe Resorcin, 2-Methylresorcin und 4-Chlorresorcin in einer Gesamtmenge von 0,10 bis 0,85 Gew.-%, bevorzugt von 0,15 bis 0,75 Gew.-%, weiter bevorzugt von 0,20 bis 0,65 Gew.-% und besonders bevorzugt von 0,25 bis 0,55 Gew.-% - bezogen auf das Gesamtgewicht des anwendungsbereiten Mittels - enthält.

7. Anwendungsbereites Mittel nach einem der Ansprüche 1 bis 6, **dadurch gekennzeichnet, dass** die Zubereitung (A) als Oxidationsfarbstoffvorprodukt vom Kupplertyp (d) ein oder mehrere m-Aminophenolderivate ausgewählt aus der Gruppe m-Aminophenol, 5-Amino-2-methylphenol und 3-Amino-2-chlor-6-methylphenol in einer Gesamtmenge von 0,01 bis 0,55 Gew.-%, bevorzugt von 0,03 bis 0,45 Gew.-%, weiter bevorzugt von 0,05 bis 0,35 und besonders bevorzugt von 0,07 bis 0,25 Gew.-% - bezogen auf das Gesamtgewicht des anwendungsbereiten Mittels - enthält.

8. Anwendungsbereites Mittel nach einem der Ansprüche 1 bis 7, **dadurch gekennzeichnet, dass** die Zubereitung (A) als Oxidationsfarbstoffvorprodukt vom Kupplertyp (d) ein oder mehrere 2-Aminopyridinderivate ausgewählt aus der Gruppe 3-Amino-2-methylamino-6-methoxypyridin und 2-Amino-3-hydroxypyridin in einer Gesamtmenge von 0,001 bis 0,35 Gew.-%, bevorzugt von 0,005 bis 0,25 Gew.-%, weiter bevorzugt von 0,01 bis 0,15 Gew.-% und besonders bevorzugt von 0,02 bis 0,05 Gew.-% - bezogen auf das Gesamtgewicht des anwendungsbereiten Mittels - enthält.

9. Anwendungsbereites Mittel nach einem der Ansprüche 1 bis 8, **dadurch gekennzeichnet, dass** das Mengenverhältnis aller im anwendungsbereiten Mittel enthaltenen Oxidationsfarbstoffvorprodukte zu allen im anwendungsbereiten Mittel enthaltenen Alkylglucosiden bei mindestens 1:1, bevorzugt bei mindestens 1:2 und besonders bevorzugt bei mindestens 1:3 liegt.

10. Anwendungsbereites Mittel nach einem der Ansprüche 1 bis 9, **dadurch gekennzeichnet, dass** die Zubereitung (A) als Oxidationsfarbstoffvorprodukte vom Kupplertyp (d) Resorcin und m-Aminophenol enthält.

11. Anwendungsbereites Mittel nach einem der Ansprüche 1 bis 10, **dadurch gekennzeichnet, dass** die Zubereitung (A) als Oxidationsfarbstoffvorprodukte vom Kupplertyp (d) Resorcin und 3-Amino-2-methylamino-6-methoxypyridin enthält.

12. Anwendungsbereites Mittel nach einem der Ansprüche 1 bis 11, **dadurch gekennzeichnet, dass** die Zubereitung (A) als Oxidationsfarbstoffvorprodukte vom Kupplertyp (d) m-Aminophenol und 3-Amino-2-methylamino-6-methoxypyridin enthält.

13. Anwendungsbereites Mittel nach einem der Ansprüche 1 bis 12, **dadurch gekennzeichnet, dass** die Zubereitung (A) als Oxidationsfarbstoffvorprodukte vom Kupplertyp (d) Resorcin, m-Aminophenol und 3-Amino-2-methylamino-6-methoxypyridin enthält.

14. Anwendungsbereites Mittel nach einem der Ansprüche 1 bis 13, **dadurch gekennzeichnet, dass**
- das durch Vermischen der Zubereitungen (A) und (B) hergestellte anwendungsbereite Mittel Wasserstoffperoxid in einer Menge von 1,5 bis 6,5 Gew.-%, weiter bevorzugt 2,2 bis 4,5 Gew.-% und besonders bevorzugt 3,2 bis 3,8 Gew.-% (berechnet als 100 %-iges Wasserstoffperoxid) - bezogen auf das Gesamtgewicht des anwendungsbereiten Mittels - enthält.

15. Verwendung der Kombination aus
(a) mindestens einem ersten Alkylglucosid der Formel (I) 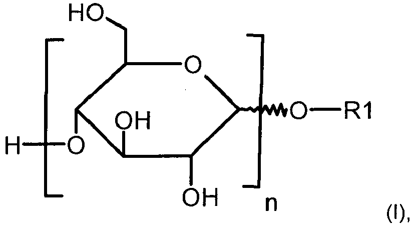
worin R1 für eine unverzweigte oder verzweigte, gesättigte oder ungesättigte C₂₀-C₂₈-Alkylgruppe steht und
n für eine ganze Zahl von 1 bis 10 steht und
(b) mindestens einem zweiten Alkylglucosid der Formel (II) 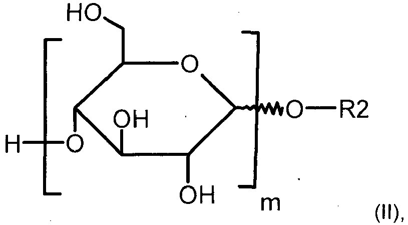
worin R2 für eine unverzweigte oder verzweigte, gesättigte oder ungesättigte C₈-C₁₈-Alkylgruppe steht und
m für eine ganze Zahl von 1 bis 10 steht,
zur Verhinderung der durch atmosphärischen Sauerstoff hervorgerufenen Verfärbung von Mitteln zum oxidativen Färben und/oder Aufhellen von keratinischen Fasern, welche in einem kosmetischen Träger
(c) mindestens ein Oxidationsfarbstoffvorprodukt vom Entwicklertyp und
(d) mindestens ein Oxidationsfarbstoffvorprodukt vom Kupplertyp enthalten.

## Claims

1. A ready-to-apply agent for dyeing and/or lightening keratin fibers, **characterized in that** it is prepared immediately before application by mixing preparations (A) and (B),
- preparation (A) being an agent containing, in a cosmetic carrier,
(a) one or more alkyl glucosides of formula (I) in a total amount of from 0.3 to 4.5 wt.%, based on the total weight of the ready-to-apply agent 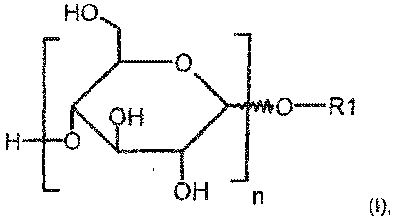
where R1 represents an unbranched or branched, saturated or unsaturated C₂₀-C₂₈ alkyl group, and
n represents an integer from 1 to 10,
(b) at least one second alkyl glucoside of formula (II) 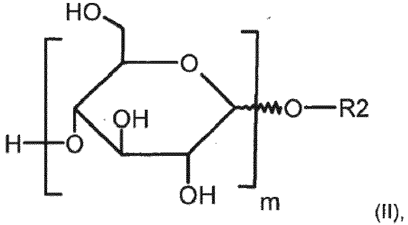
where R2 represents an unbranched or branched, saturated or unsaturated C₈-C₁₈ alkyl group, and
m represents an integer from 1 to 10,
(c) at least one developer-type oxidation dye precursor, and
(d) at least one coupler-type oxidation dye precursor, and
- preparation (B) being an agent containing, in a cosmetic carrier,
hydrogen peroxide, and
- the ready-to-apply agent prepared by mixing preparations (A) and (B) containing hydrogen peroxide in an amount of from 0.5 to 8.0 wt.% (calculated as 100% hydrogen peroxide), based on the total weight of the ready-to-apply agent.

2. The ready-to-apply agent according to claim 1, **characterized in that** preparation (A) contains one or more alkyl glucosides (a) of formula (I) in a total amount of from 0.5 to 3.5 wt.%, preferably from 0.7 to 2.5 wt.%, and particularly preferably from 0.9 to 1.5 wt.%, based on the total weight of the ready-to-apply agent.

3. The ready-to-apply agent according to one of claims 1 or 2, **characterized in that** preparation (A) contains one or more alkyl glucosides (b) of formula (II) in a total amount of from 1.2 to 8.0 wt.%, preferably from 1.6 to 6.5 wt.%, more preferably from 2.0 to 5.0 wt.%, and particularly preferably from 2.4 to 3.5 wt.%, based on the total weight of the ready-to-apply agent.

4. The ready-to-apply agent according to one of claims 1 to 3, **characterized in that** the amount ratio of all alkyl glucosides of formula (I) contained in the ready-to-apply agent to all alkyl glucosides of formula (II) contained in the ready-to-apply agent is from 1:2 to 1:10, preferably from 1:2 to 1:5.

5. The ready-to-apply agent according to one of claims 1 to 4, **characterized in that** preparation (A) contains p-toluylenediamine in the form of a sulfate salt thereof as a developer-type oxidation dye precursor (c) in an amount of from 0.45 to 1.45 wt.%, preferably from 0.55 to 1.35 wt.%, more preferably from 0.65 to 1.25 wt.%, and particularly preferably from 0.70 to 1.05 wt.%, based on the total weight of the ready-to-apply agent.

6. The ready-to-apply agent according to one of claims 1 to 5, **characterized in that** preparation (A) contains one or more resorcinol derivatives as a coupler-type oxidation dye precursor (d), which derivatives are selected from the group of resorcinol, 2-methylresorcinol and 4-chlororesorcinol, in a total amount of from 0.10 to 0.85 wt.%, preferably from 0.15 to 0.75 wt.%, more preferably from 0.20 to 0.65 wt.%, and particularly preferably from 0.25 to 0.55 wt.%, based on the total weight of the ready-to-apply agent.

7. The ready-to-apply agent according to one of claims 1 to 6, **characterized in that** preparation (A) contains one or more m-amino-phenol derivatives as a coupler-type oxidation dye precursor (d), which derivatives are selected from the group of m-aminophenol, 5-amino-2-methylphenol and 3-amino-2-chloro-6-methylphenol, in a total amount of from 0.01 to 0.55 wt.%, preferably from 0.03 to 0.45 wt.%, more preferably from 0.05 to 0.35 wt.%, and particularly preferably from 0.07 to 0.25 wt.%, based on the total weight of the ready-to-apply agent.

8. The ready-to-apply agent according to one of claims 1 to 7, **characterized in that** preparation (A) contains one or more 2-amino-pyridine derivatives as a coupler-type oxidation dye precursor (d), which derivatives are selected from the group of 3-amino-2-methylamino-6-methoxypyridine and 2-amino-3-hydroxypyridine, in a total amount of from 0.001 to 0.35 wt.%, preferably from 0.005 to 0.25 wt.%, more preferably from 0.01 to 0.15 wt.%, and particularly preferably from 0.02 to 0.05 wt.%, based on the total weight of the ready-to-apply agent.

9. The ready-to-apply agent according to one of claims 1 to 8, **characterized in that** the amount ratio of all oxidation dye precursors contained in the ready-to-apply agent to all alkyl glucosides contained in the ready-to-apply agent is at least 1:1, preferably at least 1:2, and particularly preferably at least 1:3.

10. The ready-to-apply agent according to one of claims 1 to 9, **characterized in that** preparation (A) contains resorcinol and m-aminophenol as a coupler-type oxidation dye precursor (d).

11. The ready-to-apply agent according to one of claims 1 to 10, **characterized in that** preparation (A) contains resorcinol and 3-amino-2-methylamino-6-methoxypyridine as a coupler-type oxidation dye precursor (d).

12. The ready-to-apply agent according to one of claims 1 to 11, **characterized in that** preparation (A) contains m-aminophenol and 3-amino-2-methylamino-6-methoxypyridine as a coupler-type oxidation dye precursor (d).

13. The ready-to-apply agent according to one of claims 1 to 12, **characterized in that** preparation (A) contains resorcinol, m-aminophenol and 3-amino-2-methylamino-6-methoxypyridine as a coupler-type oxidation dye precursor (d).

14. The ready-to-apply agent according to one of claims 1 to 13, **characterized in that**
- the ready-to-apply agent prepared by mixing preparations (A) and (B) contains hydrogen peroxide in an amount of from 1.5 to 6.5 wt.%, more preferably from 2.2 to 4.5 wt.%, and particularly preferably from 3.2 to 3.8 wt.% (calculated as 100% hydrogen peroxide), based on the total weight of the ready-to-apply agent.

15. The use of the combination of
(a) at least one first alkyl glucoside of formula (I), 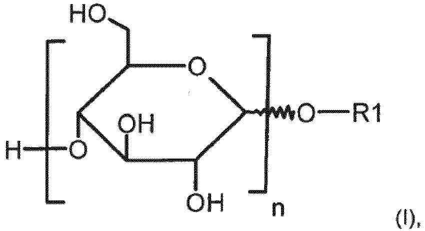
where R1 represents an unbranched or branched, saturated or unsaturated C₂₀-C₂₈ alkyl group, and
n represents an integer from 1 to 10, and
(b) at least one second alkyl glucoside of formula (II) 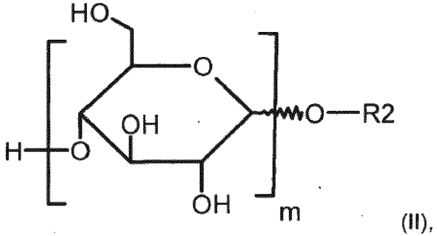
where R2 represents an unbranched or branched, saturated or unsaturated C₈-C₁₈ alkyl group, and
m represents an integer from 1 to 10,
for preventing discoloration of agents for oxidatively dyeing and/or lightening keratin fibers caused by atmospheric oxygen, which agents contain, in a cosmetic carrier,
(c) at least one developer-type oxidation dye precursor, and
(d) at least one coupler-type oxidation dye precursor.

## Revendications

1. Agent prêt à l'emploi de coloration et/ou d'éclaircissement de fibres de kératine, **caractérisé en ce qu'**il est produit, immédiatement avant l'utilisation, par mélange des préparations (A) et (B),
- la préparation (A) étant un agent qui contient dans un support cosmétique
(a) un ou plusieurs alkylglucosides de la formule (I) dans une quantité totale de 0,3 à 4,5 % en poids, par rapport au poids total de l'agent prêt à l'emploi, 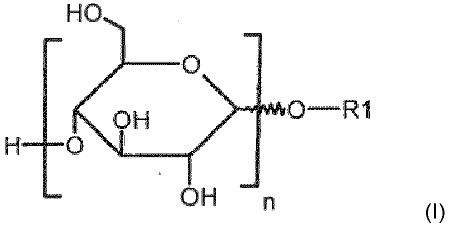
formule dans laquelle R1 représente un groupe alkyle en C₂₀ à C₂₈ non ramifié ou ramifié, saturé ou insaturé et
n est un nombre entier de 1 à 10,
(b) au moins un deuxième alkylglucoside de la formule (II) 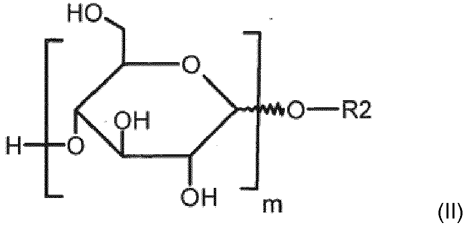
formule dans laquelle R2 représente un groupe alkyle en C₈ à C₁₈ non ramifié ou ramifié, saturé ou insaturé et
m est un nombre entier de 1 à 10,
(c) au moins un précurseur de colorant d'oxydation du type développeur et
(d) au moins un précurseur de colorant d'oxydation du type copulateur, et
- la préparation (B) étant un agent qui contient du peroxyde d'hydrogène dans un support cosmétique, et
- l'agent prêt à l'emploi, produit par mélange des préparations (A) et (B), contenant du peroxyde d'hydrogène dans une quantité de 0,5 à 8,0 % en poids (calculée en tant peroxyde d'hydrogène à 100%), par rapport au poids total de l'agent prêt à l'emploi.

2. Agent prêt à l'emploi selon la revendication 1, **caractérisé en ce que** la préparation (A) contient un ou plusieurs alkylglucosides (a) de la formule (I) dans une quantité totale de 0,5 à 3,5 % en poids, de préférence de 0,7 à 2,5 % en poids et de manière particulièrement préférée de 0,9 à 1,5 % en poids, par rapport au poids total de l'agent prêt à l'emploi.

3. Agent prêt à l'emploi selon l'une des revendications 1 ou 2, **caractérisé en ce que** la préparation (A) contient un ou plusieurs alkylglucosides (b) de la formule (II) dans une quantité totale de 1,2 à 8,0 % en poids, de préférence de 1,6 à 6,5 % en poids, plus préférablement de 2,0 à 5,0 % en poids et de manière particulièrement préférée de 2,4 à 3,5 % en poids, par rapport au poids total de l'agent prêt à l'emploi.

4. Agent prêt à l'emploi selon l'une des revendications 1 à 3, **caractérisé en ce que** le rapport quantitatif de tous les alkylglucosides de la formule (I), contenus dans l'agent prêt à l'emploi, à tous les alkylglucosides de la formule (II) est de 1:2 à 1:10, de préférence de 1:2 à 1:5.

5. Agent prêt à l'emploi selon l'une des revendications 1 à 4, **caractérisé en ce que** la préparation (A) contient comme précurseur de colorant d'oxydation du type développeur (c) de la p-toluènediamine, qui se présente sous la forme de son sel sulfate, dans une quantité de 0,45 à 1,45 % en poids, de préférence de 0,55 à 1,35 % en poids, plus préférablement de 0,65 à 1,25 % en poids et de manière particulièrement préférée de 0,70 à 1,05 % en poids, par rapport au poids total de l'agent prêt à l'emploi.

6. Agent prêt à l'emploi selon l'une des revendications 1 à 5, **caractérisé en ce que** la préparation (A) contient comme précurseur de colorant d'oxydation du type copulateur (d) un ou plusieurs dérivés du résorcinol choisis dans le groupe comportant le résorcinol, le 2-méthylrésorcinol et le 4-chlororésorcinol dans une quantité totale de 0,10 à 0,85 % en poids, de préférence de 0,15 à 0,75 % en poids, plus préférablement de 0,20 à 0,65 % en poids et de manière particulièrement préférée de 0,25 à 0,55 % en poids, par rapport au poids total de l'agent prêt à l'emploi.

7. Agent utilisable selon l'une des revendications 1 à 6, **caractérisé en ce que** la préparation (A) contient comme précurseur de colorant d'oxydation du type copulateur (d) un ou plusieurs dérivés du m-aminophénol choisis dans le groupe comportant le m-aminophénol, le 5-amino-2-méthylphénol et le 3-amino-2-chloro-6-méthylphénol dans une quantité totale de 0,01 à 0,55 % en poids, de préférence de 0,03 à 0,45 % en poids, plus préférablement de 0,05 à 0,35 % en poids et de manière particulièrement préférée de 0,07 à 0,25 % en poids par rapport au poids total de l'agent prêt à l'emploi.

8. Agent prêt à l'emploi selon l'une des revendications 1 à 7, **caractérisé en ce que** la préparation (A) contient comme précurseur de colorant d'oxydation du type copulateur (d) un ou plusieurs dérivés de la 2-amino-pyridine choisis dans le groupe comportant la 3-amino-2-méthylamino-6-méthoxypyridine et la 2-amino-3-hydroxypyridine dans une quantité totale de 0,001 à 0,35 % en poids, de préférence de 0,005 à 0,25 % en poids, plus préférablement de 0,01 à 0,15 % en poids, et de manière particulièrement préférée de 0,02 à 0,05 % en poids, par rapport au poids total de l'agent prêt à l'emploi.

9. Agent prêt à l'emploi selon l'une des revendications 1 à 8, **caractérisé en ce que** le rapport quantitatif de tous les précurseurs de colorants d'oxydation contenus dans l'agent prêt à l'emploi à tous les alkylglucosides contenus dans l'agent prêt à l'emploi est d'au moins 1:1 et de préférence d'au moins 1:2 et de manière particulièrement préférée d'au moins 1:3.

10. Agent prêt à l'emploi selon l'une des revendications 1 à 9, **caractérisé en ce que** la préparation (A) contient, comme précurseurs de colorants d'oxydation de type copulateur (d), du résorcinol et du m-aminophénol.

11. Agent prêt à l'emploi selon l'une des revendications 1 à 10, **caractérisé en ce que** la préparation (A) contient comme précurseurs de colorant d'oxydation du type copulateur (d) du résorcinol et de la 3-amino-2-méthylamino-6-méthoxypyridine.

12. Agent prêt à l'emploi selon l'une des revendications 1 à 11, **caractérisé en ce que** la préparation (A) contient comme précurseurs de colorant d'oxydation du type copulateur (d) du m-aminophénol et de la 3-amino-2-méthylamino-6-méthoxypyridine.

13. Agent prêt à l'emploi selon l'une des revendications 1 à 12, **caractérisé en ce que** la préparation (A) contient comme précurseurs de colorant d'oxydation du type copulateur (d) du résorcinol, du m-aminophénol et de la 3-amino-2-méthylamino-6-méthoxypyridine.

14. Agent prêt à l'emploi selon l'une des revendications 1 à 13, **caractérisé en ce que**
- l'agent prêt à l'emploi, produit par mélange des préparations (A) et (B), est du peroxyde d'hydrogène dans une quantité de 1,5 à 6,5 % en poids, plus préférablement de 2,2 à 4,5 % en poids et de manière particulièrement préférée 3,2 à 3,8 % en poids (calculée en tant que peroxyde d'hydrogène à 100%), par rapport au poids total de l'agent prêt à l'emploi.

15. Utilisation de la combinaison
(a) d'au moins un premier alkylglucoside de la formule (I) 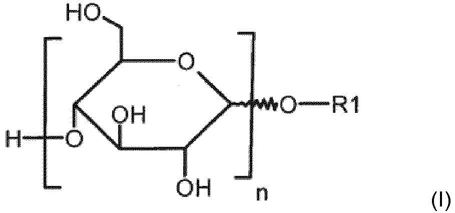
formule dans laquelle R1 représente un groupe alkyle en C₂₀ à C₂₈ non ramifié ou ramifié, saturé ou insaturé et
n est un nombre entier de 1 à 10 et
(b) d'au moins un deuxième alkylglucoside de la formule (II) 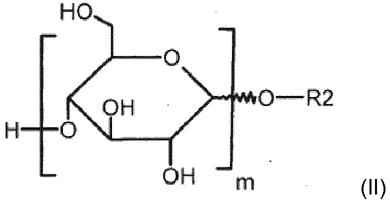
formule dans laquelle R2 représente un groupe alkyle en C₈ à C₁₈ non ramifié ou ramifié, saturé ou insaturé, et
m est un nombre entier de 1 à 10,
pour empêcher la décoloration, provoquée par l'oxygène atmosphérique, d'agents de coloration et/ou d'éclaircissement par oxydation de fibres de kératine, lesquels agents contiennent dans un support cosmétique
(c) au moins un précurseur de colorant d'oxydation du type développeur et
(d) au moins un précurseur de colorant d'oxydation du type copulateur.
